(19) Europäisches Patentamt
European Patent Office
Office européen des brevets

(11) Numéro de publication: **0 001 944**
**B1**

# (12) FASCICULE DE BREVET EUROPEEN

(45) Date de publication du fascicule de brevet: **25.11.81**

(21) Numéro de dépôt: **78400142.2**

(22) Date de dépôt: **20.10.78**

(51) Int. Cl.³: **C 07 C 69/74,**
C 07 C 69/76,
C 07 C 67/14,
C 07 C 67/10,
C 07 C 121/75,
C 07 C 120/06,
A 01 N 25/00

## (54) Esters d'alcools alpha-halogénés, procédé de préparation et application à la synthèse d'esters d'alcools alpha-cyanés.

(30) Priorité: **27.10.77 FR 7732414**
**27.10.77 FR 7732415**
**24.07.78 FR 7821811**
**24.07.78 FR 7821812**

(43) Date de publication de la demande:
**16.05.79 Bulletin 79/10**

(45) Mention de la délivrance du brevet:
**25.11.81 Bulletin 81/47**

(84) Etats Contractants Désignés:
**BE CH DE GB LU NL**

(56) Documents cités:
**DE - A - 2 437 882**
**FR - A - 2 364 884**
**NL - A - 7 609 841**

(73) Titulaire: **ROUSSEL-UCLAF**
**102, route de Noisy Boîte postale no.9**
**F-93230 Romainville (FR)**

(72) Inventeur: **Martel, Jacques**
**15, ure Douvillez**
**F-93140 Bondy (FR)**
Inventeur: **Tessier, Jean**
**30, rue Jean Moulin**
**F-94300 Vincennes (FR)**
Inventeur: **Demoute, Jean-Pierre**
**249 bis, rue de Rosny**
**F-93100 Montreuil Sous Bois (FR)**
Inventeur: **Teche, André**
**51, Avenue Joliot Curie**
**F-92000 Nanterre (FR)**

(74) Mandataire: **Burlot, Pierre et al,**
**Boite postale no 9 102, route de Noisy**
**F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 001 944 B1**

Esters d'alcools $\alpha$-halogénés, procédé de préparation et application à la synthèse d'esters d'alcools $\alpha$-cyanés

La présente invention a pour objet, sous toutes leurs formes stéréoisomères ou sous forme de mélange de stéréoisomères, les composés de formule générale $I_A$:

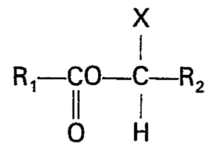

($I_A$)

dans laquelle:

— X représente un atome de fluor, de chlore ou de brome,

— $R_1$ représente:

*soit* un groupement $R_1'$

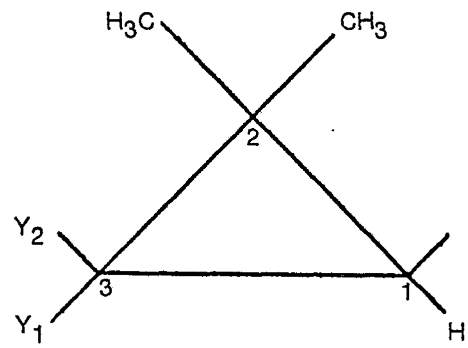

($R_1'$)

dans lequel

*ou bien* $Y_1$ et $Y_2$, sont identiques et représentent un radical méthyle,

*ou bien* $Y_1$ représente un atome d'hydrogène et $Y_2$ représente un groupement $Y_2'$:

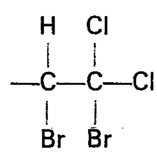

($Y_2'$)

*ou bien* $Y_1$ représente un atome d'hydrogène et $Y_2$ représente un groupement:

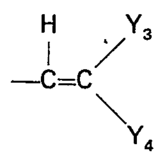

$Y_3$ et $Y_4$, identiques ou différents, représentant un atome de fluor, de chlore ou de brome, ou bien $Y_3$ et $Y_4$, identiques ou différents, représentant un radical méthyle ou un atome d'hydrogène, le groupement $R_1'$ correspondant, dans le cas où $Y_2$ représente un groupement:

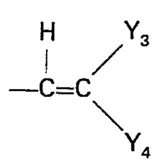

à un reste d'acide de structure cis ou trans, racémique ou optiquement actif, ou à un mélange de restes d'acide de structure cis et d'acide de structure trans,

*soit* un groupement ($R_1''$):

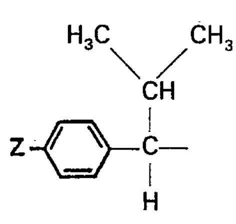

($R_1''$)

dans lequel Z représente un atome d'hydrogène, un atome de fluor, de chlore ou de brome, un radical alcoyle, linéaire ou ramifié, comportant de 1 à 4 atomes de carbone, un radical alcoyloxy, linéaire ou ramifié, comportant de 1 à 4 atomes de carbone, le groupement ($R_1''$) correspondant à un reste d'acide racémique ou optiquement actif,

$R_2$ représente, dans le cas où $Y_2$ est différent de $Y_2'$:

*ou bien* un reste choisi parmi les groupements suivants:

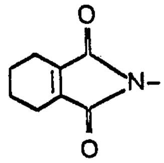, 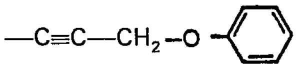, 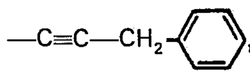, 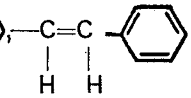, 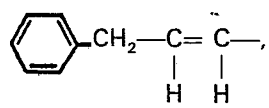,

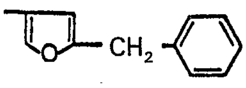 *ou bien* le reste 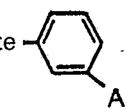 dans lequel A représente soit un groupement:

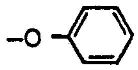 soit un groupement 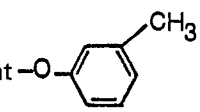 soit un groupement: 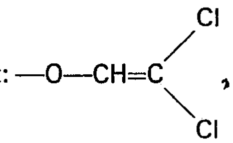,

soit un groupement 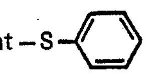 soit un groupement 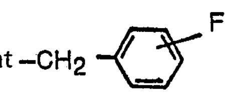

ou $R_2$ représente, dans le cas où $Y_2$ représente un groupe $Y_2'$, le reste 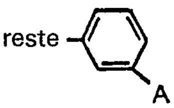

dans lequel A représente un groupement 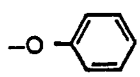

L'invention a notamment pour objet, sous toutes leurs formes stéréoisomères, les composés de formule générale I:

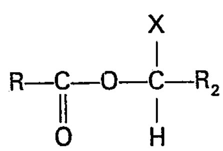 (I)

dans laquelle $R_2$ et X sont définis comme précédemment, et R représente *soit* un groupement R':

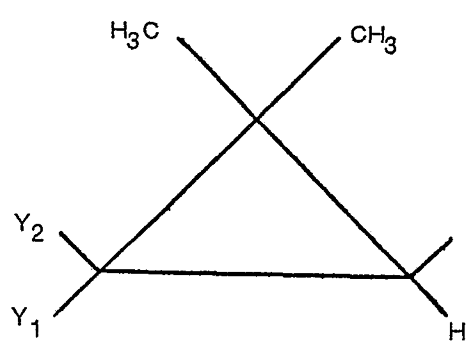 (R')

dans lequel:

*ou bien* $Y_1$ et $Y_2$, sont identiques et représentent un radical méthyle,

*ou bien* $Y_1$ représente un atome d'hydrogène et $Y_2$ représente un groupement:

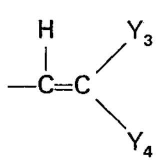

$Y_3$ et $Y_4$, identiques ou différents, représentant un atome de fluor, de chlore ou de brome, ou bien $Y_3$ et $Y_4$, identiques ou différents, représentant un radical méthyle ou un atome d'hydrogène, le groupement $R_1'$ correspondant, dans le cas où $Y_2$ représente un groupement:

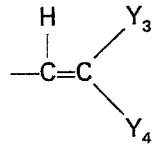

à un reste d'acide de structure cis ou trans, racémique ou optiquement actif, ou à un mélange de restes d'acide de structure cis et d'acide de structure trans.

— *soit* un groupement $R_1''$ tel que défini précédemment.

Les composés ($I_A$) existent sous de nombreuses formés stéréoisomères.

Lorsque, dans le groupement $R_1'$, $Y_1$ représente un atome d'hydrogène et $Y_2$ représente un groupement:

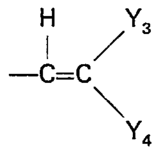

le groupement $R_1$ possède deux carbones asymétriques en positions 1 et 3 du cycle cyclopropanique. Les acides cyclopropane carboxyliques correspondants peuvent être alors de structure cis ou de structure trans, racémiques ou optiquement actifs, ou peuvent être des mélanges d'acides de structure cis et d'acides de structure trans.

Dans le cas où $Y_3$ est différent de $Y_4$ il existe, de plus, une isomérie (E) et (Z) au niveau de la double liaison.

Le groupement $R_1''$ dérive d'acides 2-aryl 2-isopropyl acétiques substitués dont le carbone en position 2 est un carbone asymétrique, ce qui entraîne dans ce groupement l'existence de deux formes énantiomères et d'un racémique.

De plus, dans les composés de formule $I_A$; le carbone porteur du substituant X est un carbone asymétrique.

On peut considérer comme vraisemblable que, d'après le procédé d'obtention des composés de formule $I_A$, dans le cas où le groupement $R_1$ comporte un ou plusieurs carbones asymétriques de configuration absolue bien définie, il y ait une induction asymétrique au niveau du carbone porteur de X. Mais l'induction ainsi obtenue n'est, en général, pas totale et le centre chiral (porteur de X) n'est, en général, ni entièrement de structure (R), ni entièrement de structure (S). Il en résulte que le produit $I_A$ obtenu, dans ce cas, est un mélange de deux diastéréoisomères que l'on peut séparer. Ces diastéréoisomères seront appelés par la suite, isomère A et isomère B.

Par convention, l'isomère A est celui des deux qui est le plus mobile en chromatographie sur couche mince. D'après certaines considérations théoriques mais sans vouloir être lié par l'exactitude de l'attribution des chiralités que l'on en déduit, il est plausible d'attribuer à l'isomère A, la configuration absolue (S) pour le carbone porteur du substituant X.

L'atome de carbone asymétrique de la chaîne latérale éthylique des composés de formule $I_A$ dans laquelle $R_1$ représente un groupement $R_1'$ dans lequel $Y_1$ représente un atome d'hydrogène et $Y_2$ représente un groupement

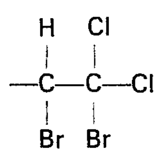

entraîne, pour sa part, l'existence de deux diastéréoisomères que l'on peut séparer, par exemple, par chromatographie.

Les composés de formule $I_A$ de l'invention, englobent pour une définition donnée des substituants X, $R_1$ et $R_2$, tous les composés provenant de la combinaison d'un isomère, optiquement actif (ou racémique), résultant de l'existence du ou des carbones asymétriques de la partie acide $R_1$ de la molécule avec un isomère optiquement actif (ou racémique) correspondant à la copule alcoolique:

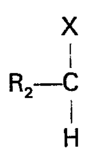

L'invention a notamment pour objet les composés de formule I comportant un groupement (R') dans lequel $Y_1$ représente un atome d'hydrogène et $Y_2$ représente un groupement:

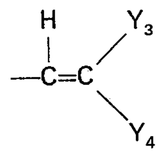

Parmi les composés, objet de l'invention, on citera plus particulièrement ceux dans lesquels $R_2$ représente un groupement

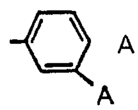 A ayant pour valeur 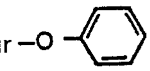

ceux dans lesquels X représente un atome de fluor; ceux dans lesquels $R_2$ représente un groupement

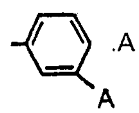 .A ayant pour valeur 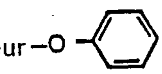

et dans lesquels X représente un atome de fluor ou de brome ainsi que ceux dans lesquels X représente un atome de chlore.

Parmi les composés de formule générale $I_A$, les produits décrits dans les exemples et notamment:

— le 1R, cis 2,2-diméthyl 3-(2',2'-dibromovinyl)cyclopropane-1-carboxylate de (RS) α-chloro 3-phénoxy benzyle,

— le 1R, cis 2,2-diméthyl 3-(2',2'-dibromovinyl)cyclopropane-1-carboxylate de (RS) α-fluoro 3-phénoxy benzyle,

— le 1R, cis 2,2-diméthyl 3-(2',2'-dichlorovinyl)cyclopropane-1-carboxylate de (RS) α-chloro 3-phénoxy benzyle,

— le 2-(parachlorophényl) 2-isopropyl acétate de (RS) α-chloro 3-phénoxy benzyle et

— le 1R, trans 2,2-diméthyl 3-(1',2'-dibromo 2' 2'-dichloroéthyl) cyclopropane 1-carboxylate de (R,S) α-chloro 3-phénoxybenzyle sont particulièrement intéressants.

L'invention a également pour objet un procédé de préparation des composés de formule générale $I_A$, telle que définie précédemment, procédé caractérisé en ce que l'on fait réagir, en présence d'un catalyseur acide, un halogénure, d'acide de formule générale II:

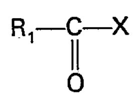 (II)

dans laquelle X et $R_1$ conservent les significations précitées avec un aldéhyde de formule générale III:

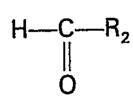 (III)

dans laquelle $R_2$ conserve les significations précitées.

L'invention a notamment pour objet un procédé tel que défini ci-dessus, pour la préparation des composés de formule générale I, telle que définie précédemment, procédé caractérisé en ce que l'on utilise au départ un halogénure d'acide de formule générale $II_A$:

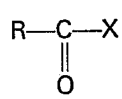 ($II_A$)

dans laquelle R et X conservent les significations précitées.

La préparation des chlorures ou des bromures d'acide de formule (II) ou ($II_A$) est effectuée par les méthodes classiques.

La préparation des fluorures d'acide de formule II est avantageusement effectuée selon la méthode décrite par MUKAIYAMA, Chem. Letters p. 303—306 (1976).

Le catalyseur acide, en présence duquel est effectué la condensation de l'halogénure d'acide II et de l'aldéhyde III est, de préférence, soit un acide de Lewis tel que le chlorure de zinc, le chlorure d'aluminium ou le chlorure ferrique, soit un acide protonique tel que l'acide paratoluène sulfonique ou l'oléum.

La condensation de l'halogénure d'acide (II) et de l'aldéhyde (III) est effectuée commodément par simple mélange des réactifs et du catalyseur acide, sans addition de solvant.

Cette condensation peut aussi être effectuée en présence d'un solvant organique.

L'invention a aussi pour objet un procédé de préparation des composés de formule générale I dans lesquels X représente un atome de fluor et dans lesquels $R_2$ représente soit un reste:

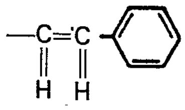 soit un reste 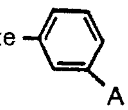

dans lequel A représente un groupement: 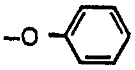,

un groupement 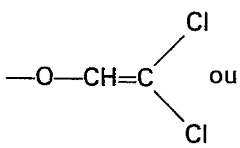 ou un groupement: 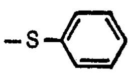

caractérisé en ce que l'on fait réagir un fluorure d'ammonium quaternaire fixé sur une résine avec un chlorure ($Z_1$) soit de formule

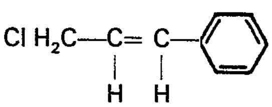 soit de formule: 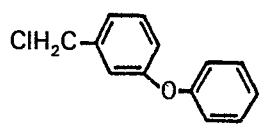

soit de formule: 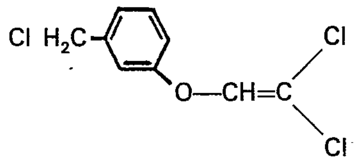

soit de formule: 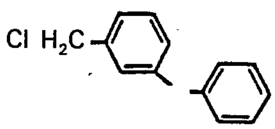

soumet, en présence d'azoisobutyronitrile, le fluorure ($Z_2$) résultant correspondant, à l'action du N-bromosuccinimide pour obtenir un dérivé mixte bromé et fluoré ($Z_3$) soit de formule:

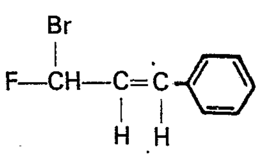 soit de formule: 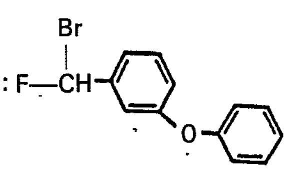

soit de formule: 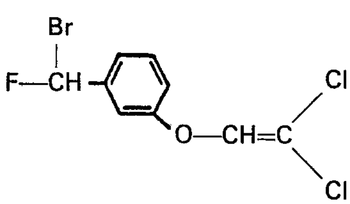 soit de formule: 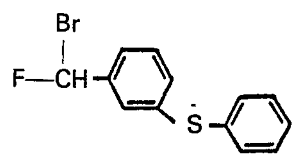

puis fait réagir ce composé avec un sel alcalin de formule générale IV:

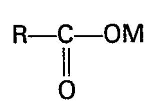 (IV)

dans laquelle R conserve les significations précitées et M représente un ion dérivant d'un métal alcalin.

Pour la préparation du fluorure ($Z_2$), on utilise avantageusement une résine du type Amberlite A-26, commercialisée par la firme ROHM et HAAS, que l'on lave par percolation avec une solution

aqueuse de soude, puis que l'on lave a l'eau jusqu'à neutralité. Le résine humide est ensuite agitée dans une solution aqueuse d'acide fluorhydrique, lavée à l'eau puis séchée par lavage avec des solvants organiques.

La fluoration du chlorure ($Z_1$) par la résine ainsi préparée est effectuée avantageusement au sein d'un solvant organique tel que le toluène.

L'obtention du dérivé mixte bromé et fluoré ($Z_3$) par condensation du fluorure $Z_2$ et du N-bromosuccinimide, en présence d'azoisobutyronitrile est commodément effectuée au sein du tétrachlorure de carbone en opérant au reflux.

La condensation du dérivé mixte bromé et fluoré ($Z_3$) et du sel de sodium de l'acide IV est avantageusement effectuée au sein d'un solvant organique tel que le diméthylformamide.

Les composés de formule $I_A$, telle que définie précédemment, se sont avérés être doués d'une activité insecticide intéressante et peuvent être utilisés comme insecticides dans les domaines domestique et agricole.

Cette activité insecticide s'est révélée particulièrement remarquable pour les composés de formule $I_A$ dans laquelle $R_2$ représente un groupement

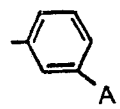

A étant un radical phénoxy.

Les composés de formule $I_A$ peuvent être utilisés pour préparer, selon les méthodes usuelles, des compositions insecticides. Ces compositions contiennent, de préférence, de 0,05 à 10% en poids de matière active et, en générale, un véhicule et/ou un agent tensio actif non ionique.

Les composés de formule $I_A$, telle que définie précédemment, sont des intermédiaires utiles dans la synthèse d'esters possédant de remarquables propriétés pesticides, notamment insecticides.

L'invention a ainsi également pour objet l'application des composés de formule $I_A$, telle que définie précédemment, à la préparation, sous toutes leurs formes stéréoisomères ou sous forme de mélange de stéréoisomères, des composés de formule $I_B$:

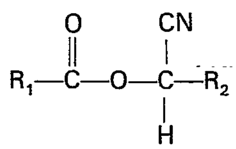

($I_B$)

dans laquelle $R_1$ et $R_2$ conservent les mêmes significations que dans la formule $I_A$, application caractérisée en ce que l'on fait réagir un composé générateur d'ions $CN^-$ sur un composé de formule générale $I_A$:

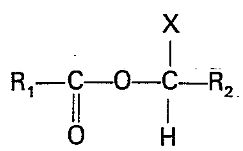

($I_A$).

dans laquelle X, $R_1$ et $R_2$ conservent les significations précitées, les composés $I_B$ résultants existant sous les formes stéréoisomères correspondant aux formes stéréoisomères des composés $I_A$ utilisés.

L'invention a notamment pour objet une application des composés de formule I, telle que définie ci-dessus, à la préparation des produits de formule $I_C$:

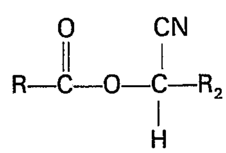

($I_C$)

dans laquelle R et $R_2$ sont définis comme précédemment, application caractérisée en ce que l'on fait réagir un composé générateur d'ions $CN^-$ sur un composé de formule générale I.

Le procédé est particulièrement avantageux lorsque, dans la formule $I_A$ ou I, X représente un atome de fluor ou de chlore.

La condensation du composé de formule générale $I_A$ ou I avec l'agent générateur d'ions $CN^-$ est effectuée commodément au sein d'un solvant organique anhydre, choisi dans le groupe constitué par l'acétonitrile et le diméthylformamide.

L'agent générateur d'ions $CN^-$ que l'on fait agir sur le composé de formule $I_A$ ou I est, de préférence, un cyanure alcalin, tel que le cyanure de sodium ou de potassium.

On peut également utiliser, avantageusement, comme agent de cyanuration, le cyanure de tétraéthyl ammonium, le 1-méthyl 1-éthyl éthanonitrile, le cyanure cuivreux.

Les produits de formule, $I_B$ ou $I_C$ obtenus sous forme de mélanges d'isomères, notamment au niveau du carbone porteur du groupement —CN, peuvent être séparés en leurs différents constituants par des méthodes connues.

L'invention a plus particulièrement pour objet, une application à la préparation, selon la méthode précitée, du 1R, cis 2,2-diméthyl 3-(2',2'-dibromovinyl) cyclopropane-1-carboxylate de RS $\alpha$-cyano 3-phénoxy benzyle, caractérisée en ce que le composé de départ utilisé est le 1R, cis 2,2-diméthyl 3-(2',2'-dibromovinyl) cyclopropane-1-carboxylate de RS $\alpha$-chloro 3-phénoxy benzyle, une application à la préparation, selon la méthode précitée, du 1R, cis 2,2-diméthyl 3-(2',2'-dibromovinyl) cyclopropane-1-carboxylate de RS $\alpha$-cyano 3-phénoxy benzyle, caractérisée en ce que le composé de départ utilisé est le 1R, cis 2,2-diméthyl 3-(2',2'-dibromovinyl) cyclopropane.1-carboxylate de RS $\alpha$-fluoro 3-phénoxy benzyle, une application à la préparation, selon la méthode précitée, du 1R, cis 2,2-diméthyl 3-(2',2'-dichlorovinyl) cyclopropane 1-carboxylate de RS $\alpha$-cyano 3-phénoxy benzyle, caractérisée en ce que le composé de départ utilisé est le 1R, cis 2,2-diméthyl 3-(2',2'-dichlorovinyl) cyclopropane 1-carboxylate de RS $\alpha$-chloro 3-phénoxy benzyle, une application à la préparation, selon la méthode précitée, du 2-parachlorophényl 2-isopropyl acétate de RS $\alpha$-cyano 3-phénoxy benzyle, caractérisé en ce que le composé de départ utilisé est le 2-parachlorophényl 2-isopropyl acétate de RS $\alpha$-chloro 3-phénoxy benzyle, une application à la préparation, selon la méthode précitée, du 1R, trans 2,2-diméthyl 3-(1',2'-dibromo 2,2'-dichloroéthyl) cyclopropane 1-carboxylate de (R,S) $\alpha$-cyano 3-phénoxy benzyle, caractérisée en ce que le composé de départ utilisé est le 1R, trans 2,2-diméthyl 3-(1',2'-dibromo 2',2'-dichloroéthyl) cyclopropane 1-carboxylate de (R,S) $\alpha$-chloro ou $\alpha$-fluoro 3-phénoxy benzyle.

De nombreux composés de formule $I_B$ sont bien connus pour leurs propriétés insecticides particulièrement intenses (voir notamment le brevet français 2 185 612 et la demande française publiée sous le n° 2 364 884).

On peut citer à ce sujet, le 1R, cis 2,2-diméthyl 3-(2'2'-dibromovinyl) cyclopropane 1-carboxylate de RS $\alpha$-cyano 3-phénoxy benzyle, le 1R, cis 2,2-diméthyl 3-(2',2'-dichlorovinyl) cyclopropane 1-carboxylate de RS $\alpha$-cyano 3-phénoxy benzyle ou le 1R, trans 2,2-diméthyl 3-(1',2'-dibromo 2',2'-dichloroéthyl) cyclopropane 1-carboxylate de R,S $\alpha$-cyano 3-phénoxy benzyle.

Le procédé de préparation des composés de formule $I_A$ et l'application de ceux-ci à la préparation des composés de formule $I_B$, constituent un nouveau procédé d'accès économique aux composés $I_B$. Il présente l'avantage d'utiliser des réactifs facilement accessibles et de conduire avec des rendements élevés aux composés $I_B$.

De plus, ce procédé permet d'éviter l'utilisation de l'alcool $\alpha$-cyano 3-phénoxy benzylique, composé fragile et délicat à manipuler.

Les composés, de formule II utilisés au départ du procédé sont des composés connus. Parmi ceux-ci, les chlorures d'acide de formule générale:

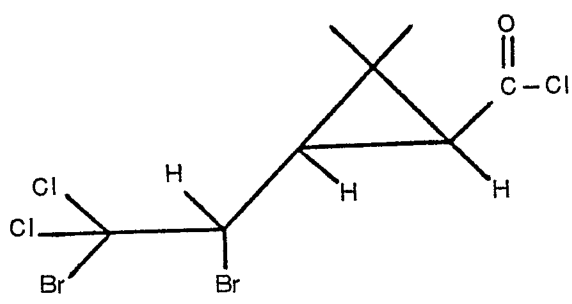

sont décrits dans la demande de brevet français 2 364 884 qui comporte également la préparation des acides correspondants. Les autres halogénures d'acide sont obtenus selon les méthodes classiques à partir des acides décrits dans la demande de brevet français 2 364 884.

Les exemples suivants illustrent l'invention sans la limiter.

Exemple 1

1R,cis 2,2-diméthyl 3-(2',2'-dibromovinyl)cycloprane-1-carboxylate de RS $\alpha$-chloro 3-phénoxy benzyle

A un mélange de 6,4 g de chlorure de l'acide 1R, cis 2,2-diméthyl 3-(2',2'-dibromovinyl)cyclopropane-1-carboxylique et de 4 g d'aldéhyde métaphénoxy benzoïque, on ajoute ensuite 10 mg de chlorure de zinc fondu (sous forme de poudre). On note un échauffement et un épaississement du milieu. On laisse encore une heure en contact à 20°C et dans des conditions rigoureusement anhydres. On obtient le 1R,cis 2,2-diméthyl 3-(2',2'-dibromovinyl)cyclopropane-1-carboxylate de RS $\alpha$-chloro-3-phénoxy benzyle brut.

*Spectre Infra-Rouge* (chloroforme)
Absorption à 1749 $cm^{-1}$ caractéristique de C=O.

*Spectre de RMN* (deutéro chloroforme)
Pics à 1,26—1,33 p.p.m. caractéristiques des hydrogènes des méthyles géminés,
pics à 1,85—2,10 p.p.m. caractéristiques des hydrogènes du cyclopropyle;
pics à 6,76—6,90 p.p.m. caractéristiques de l'hydrogène éthylénique;
pics à 7,0 p.p.m. et 7,66 p.p.m. caractéristiques des hydrogènes du noyau aromatique et benzylique.

Le chlorure de l'acide 1R,cis 2,2-diméthyl 3-(2',2'-dibromovinyl)cyclopropane-1-carboxylique peut être obtenu d'une manière analogue à celle décrite aux exemples 16 et 17 du brevet français 2.185.612.

Exemple 2

1R,cis 2.2-dimethyl 3-(2',2'-dibromovinyl)cyclopropane-1-carboxylate de RS $\alpha$-chloro 3-phénoxy benzyle

En opérant de manière analogue à celle de l'exemple 1 et en remplaçant les 10 mg de chlorure de zinc par 60 mg de chlorure ferrique et en agitant pendant quatre heures, on obtient le 1R, cis 2,2-diméthyl 3-(2',2'-dibromovinyl)cyclopropane-1-carboxylate de RS $\alpha$-chloro 3-phénoxy benzyle brut.

Exemple 3

1R,cis 2,2-diméthyl 3-(2',2'-dibromovinyl)cyclopropane-1-carboxylate de RS $\alpha$-chloro 3-phénoxy benzyle.

En opérant de manière analogue à celle de l'exemple 1 en remplaçant les 10 mg de chlorure de zinc par 60 mg d'acide paratoluène sulfonique monohydraté et en agitant pendant vingt heures, on obtient le 1R,cis 2,2-diméthyl 3-(2',2'-dibromovinyl) cyclopropane-1-carboxylate de RS $\alpha$-chloro 3-phénoxy benzyle brut.

Exemple 4

1R,cis 2,2-diméthyl 3-(2',2'-dibromovinyl)cyclopropane-1-carboxylate de RS $\alpha$-chloro 3-phénoxy benzyle.

En opérant de manière analogue à celle de l'exemple 1 et en remplaçant les 10 mg de chlorure de zinc par une goutte d'oléum à 65 pour cent, et en agitant pendant une heuré, on obtient le 1R,cis 2,2-diméthyl 3-(2',2'-dibromovinyl)cyclopropane-1-carboxylate de RS $\alpha$-chloro 3-phénoxy benzyle brut.

Exemple 5

1R,cis 2,2-diméthyl 3-(2',2'-dibromovinyl)cyclopropane-1carboxylate de RS $\alpha$-bromo 3-phénoxy benzyle.

A un mélange de 5,66 g de bromure de l'acide 1R,cis 2,2-diméthyl 3-(2',2'-dibromovinyl)cyclopropane-1-carboxylique et de 3,11 g d'aldéhyde métaphénoxy benzoïque, on ajoute 5 mg de chlorure de zinc. On note un échauffement et un épaississement du mélange réactionnel. On laisse encore pendant une heure à 20°C et dans des conditions rigoureusement anhydres, on obtient le 1R,cis 2,2-diméthyl 3-(2',2'-dibromovinyl)cyclopropane-1-carboxylate de RS $\alpha$-bromo 3-phénoxy benzyle.

*Spectre Infra-Rouge* (chloroforme)
Absorption à 1750 $cm^{-1}$ caractéristique du carboxyle.

*Spectre de RMN* (deutéro chloroforme)
Pics à 1,26—1,33 p.p.m. caractéristiques des hydrogènes des méthyles géminés,
pics à 1,75—2,16 p.p.m. caractéristiques des hydrogènes du cyclopropyle;
pics à 6,76—6,88 p.p.m. caractéristiques de l'hydrogène éthylénique;
pics à 7,0—7,75 p.p.m. caractéristiques des hydrogènes du phényle et du benzyle.

Le bromure de l'acide 1R,cis 2,2-diméthyl 3-(2',2'-dibromovinyl)cyclopropane-1-carboxylique peut être obtenu de la manière suivante:

Dans 60 cm3 de toluène, on dissout 18 g d'acide 1R,cis 2,2-diméthyl 3-(2',2'-dibromovinyl) cyclopropane-1-carboxylique, ajoute 0,2 cm3 de pyridine puis progressivement 6 cm3 de tribromure de phosphore, agite à 20°C pendant 6 jours, sépare par décantation une huile dense qui s'est formée à la partie inférieure du mélange réactionnel, élimine le solvant par distillation sous pression réduite, rectifie le résidu et obtient 14 g de bromure de l'acide 1R,cis 2,2-dimethyl 3-(2',2'-dibromovinyl) cyclopropane-1-carboxylique (Eb. 110°C sous 0,2 mm de mercure).

*Spectre Infra-Rouge* (chloroforme)
Absorption à 1792 $cm^{-1}$ caractéristique du carboxyle.

*Spectre de RMN* (deutéro chloroforme)
Pics à 1,32—1,36 p.p.m. caractéristiques des hydrogènes des méthyles géminés;
pics à 2, 12—2, 26—2,40 p.p.m. caractéristiques de l'hydrogène en position 1 du cyclopropyle;
pics à 2,66—2,80 p.p.m. caractéristiques de l'hydrogène en position 3 du cyclopropyle;
pics à 6,53—6,66 p.p.m. caractéristiques de l'hydrogène éthylénique.

Exemple 6

1R,trans 2,2-diméthyl 3-(2',2'-dibromovinyl)cyclopropane-1-carboxylate de RS $\alpha$-chloro 3-phénoxy benzyle.

Dans un mélange de 6,4 g de chlorure de l'acide 1R,trans 2,2-diméthyl 3-(2',2'-dibromovinyl)cyclopropane-1-carboxylique et de 4 g d'aldéhyde métaphénoxy benzoïque on introduit 10 mg de chlorure de zinc, agite pendant une heure et obtient une solution de 1R,trans 2,2-diméthyl 3-(2',2'-dibromovinyl)cyclopropane-1-carboxylate de RS $\alpha$-chloro 3-phénoxy benzyle.

Exemple 7

1R,cis 2,2-diméthyl 3-(2',2'-dichlorovinyl)cyclopropane-1-carboxylate de RS $\alpha$-chloro 3-phénoxy benzyle.

Dans un mélange de 4,6 g de chlorure de l'acide 1R,cis 2,2-diméthyl 3-(2',2'-dichlorovinyl)cyclopropane-1-carboxylique et de 4 g de métaphénoxy benzaldéhyde, on introduit 10 mg de chlorure de zinc, agite pendant une heure et obtient une solution de 1R,cis 2,2-diméthyl 3-(2',2'-dichlorovinyl)cyclopropane-1-carboxylate de RS $\alpha$-chloro 3-phénoxy benzyle.

Exemple 8

2,2-diméthyl 3R-(2'-methyl 1'-propényl)cyclopropane-1R-carboxylate de RS $\alpha$-chloro 3-phénoxy benzyle.

Dans un mélange de 1,89 g de chlorure de l'acide 2,2-diméthyl 3R-(2'-méthyl 1'-propényl)cyclopropane-1R-carboxylique et de 2 g d'aldéhyde métaphénoxy benzylique, on ajoute 10 mg de chlorure de zinc, agite pendant deux heures et 30 minutes et obtient 3,89 g de 2,2-diméthyl 3R-(2'-méthyl 1'-propényl)cyclopropane-1R-carboxylate de RS $\alpha$-chloro 3-phénoxy benzyle brut.

Exemple 9

2-(parachlorophényl)2-isopropyl acétate de RS $\alpha$-chloro 3-phénoxy benzyle.

Dans un mélange de 4,7 de chlorure de l'acide 2-(parachlorophényle)2-isopropyl acétique et de 4 g de métaphénoxy benzaldéhyde on introduit 10 mg de chlorure de zinc, agite pendant trois heures à 20°C et obtient une solution de 2-(parachlorophényle)2-isopropyl acétate de RS $\alpha$-chloro 3-phénoxy benzyle.

Exemple 10

1R,cis 2.2-diméthyl 3-(2',2'-dibromovinyl)cyclopropane-1-carboxylate de RS $\alpha$-chloro cinnamyle.

Dans un mélange de 4,84 g de chlorure de l'acide 1R,cis 2,2-diméthyl 3-(2',2'-dibromovinyl)cyclopropane-1-carboxylique et de 2,22 g d'aldehyde cinnamique, on introduit 10 mg de chlorure de zinc, agite pendant une heure à 20°C et obtient 7,06 g de 1R,cis 2,2-diméthyl 3-(2',2'-dibromovinyl)cyclopropane-1-carboxylate de RS $\alpha$-chloro cinnamyle brut (F=82°C environ).

Exemple 11

1R,cis 2,2-diméthyl 3-(2',2'-dibromovinyl)cyclopropane-1-carboxylate de RS $\alpha$-fluoro 3-phénoxy benzyle.

Ce composé peut être obtenu par action du fluorure de l'acide 1R, cis 2,2-diméthyl 3-(2',2'-dibromovinyl) cyclopropane 1-carboxylique avec l'aldéhyde métaphénoxybenzoïque en présence de chlorure de zinc.

Il peut également être obtenu de la manière suivante:

*Stade A: Fluorure de métaphénoxy benzyle.*

a) *Préparation de la résine* inspirée d'un procédé décrit par G. CAINELLI et F. MANESCALCHI (Synthesis *1976* 472), 220 g de résine Amberlite A 26, de la firme Rohm et Haas sont lavés par percolation avec 1,5 litre de solution aqueuse N de soude, puis à l'eau à neutralité. La résine humide comportant des groupements $OH^-$ est agitée pendant 20 heures à 20°C dans une solution aqueuse d'acide fluorhydrique environ N, puis on essore lave abondamment à l'eau, à l'acétone et à l'éther. La résine est finalement séchée pendant 10 heures à 50°C, sous pression réduite.

b) *Obtention du fluorure de métaphénoxy benzyle.*

On chauffe à 100°C, pendant 20 heures, sous agitation un mélange de 10,5 g de chlorure de méthaphénoxy benzyle, de 150 cm3 de toluène et de 40 g de résine obtenue au paragraphe a).

On élimine la résine par filtration, la lave au chlorure de méthylène, élimine les solvants par distillation sous pression réduite, rectifie le résidu et obtient 5,8 g de fluorure de métaphénoxy benzyle (Eb. 93°C sous 0,05 mm de mercure).

*Spectre de RMN* (deutéro chloroforme)

Pics à 4,91—5,71 p.p.m. caractéristiques des hydrogènes de $—CH_2F$;

pics à 6,91—7,6 p.p.m. caractéristiques des hydrogènes des noyaux aromatiques.

*Stade B: 1-bromofluoro méthyl 3-phénoxy benzène.*

Dans 20 cm3 de tétrachlorure de carbone, on introduit 2 g de fluorure de métaphénoxy benzyle, 2 g de N-bromosuccinimide et 50 mg d'azoisobutyronitrile, porte le mélange réactionnel au reflux, maintient le reflux pendant une heure, refroidit, élimine la succinimide par filtration, concentre à sec par distillation sous pression réduite, purifie le résidu par chromatographie sur gel de silice en éluant à l'éther de pétrole (Eb. 35—75°) et obtient 1,8 g de 1-bromofluorométhyl 3-phénoxy benzène.

*Analyse:*

| | | | | |
|---|---|---|---|---|
| Calculé: | C% 55,54 | H% 3,58 | Br% 28,42 | F% 6,75 |
| Trouvé | 56,1 | 3,7 | 28,0 | 6,4. |

Spectre RMN (deutéro chloroforme)

Pics à 6,43—7,75 p.p.m. caractéristiques de l'hydrogène de

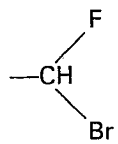

pics à 6,91—7,5 p.p.m. caractéristiques des hydrogènes des noyaux aromatiques.

*Stade C: 1R,cis 2,2-diméthyl 3-(2',2'-dibromovinyl)cycloprop-ane-1-carboxylate de RS α-fluoro 3-phénoxy benzyle.*

Dans 10 cm3 de diméthyl formamide, on introduit 1,19 g de 1-bromo-fluoro méthyl 3-phénoxy benzène, 1,5 g de sel de sodium de l'acide 1R,cis 2,2-diméthyl 3-(2',2'-dibromovinyl)cyclopropane-1-carboxylique, agite à 20°C pendant 17 heures, verse le mélange réactionnel dans l'eau glacée, extrait au benzène et aprés traitements habituels, concentre à sec par distillation sous pression réduite. Le résidu obtenu correspondant au 1R,cis 2,2-diméthyl 3-(2',2'-dibromovinyl)cyclopropane-1-carboxylate de RS α-fluoro 3-phénoxy benzyle brut est formé de deux diastéréoisomères que l'on sépare par chromatographie sur gel de silice, par élution, avec un mélange d'éther de pétrole (Eb. 35°C—75°C) et d'éther éthylique (95/5), on obtient 0,530 g du diastéréoisomère A, F=80°C le plus mobile, 0,700 g de l'autre diastéréoisomère
F=50°C le moins mobile, ainsi que 0,420 g du mélange des deux isomeres précédents, soit en tout 1,65 g de 1R,cis 2,2-diméthyl 3-(2',2'-dibromovinyl)cyclopropane-1-carboxylate de RS α-fluoro-3-phénoxy benzyle.

*L'isomère le plus mobile* (F=80°C), isomère A, posséde les caractéristiques suivants:
Spectre de RMN (deutéro chloroforme)
Pics à 1,32—1,35 p.p.m., caractéristiques des hydrogènes des méthyles géminés;
pics à 6,8—7,71 p.p.m., caractéristique de l'hydrogène porté par le même carbone que le fluor;
pics à 1,85—2,1 p.p.m. caractéristiques des hydrogènes du cyclopropyle,
pics à 6,66- p.p.m.—7,83 p.p.m. caractéristiques des hydrogènes des noyaux aromatiques.

*Dichroïsme circulaire* (dioxane)
$\Delta\varepsilon = +0{,}30$ à 284 nm (maximum),
$\Delta\varepsilon = +0{,}25$ à 278 nm (maximum);
$\Delta\varepsilon = +10{,}8$ à 215 nm (maximum).

*L'isomère le moins mobile* (F=50°C), isomère B, posséde les caractéristiques suivantes:
Spectre RMN (deutéro chloroforme)
Pics à 1,28 p.p.m. caractéristiques des hydrogènes des méthyles géminés,
pics à 6,8—7,7 p.p.m. caractéristique de l'hydrogène porté par le même carbone que le fluor;
pics à 1,86—2,25 p.p.m. caractéristiques des hydrogènes du cyclopropyle,
pics à 6,8—7,6 p.p.m. caractéristiques des hydrogènes des noyaux aromatiques.

*Dichroïsme circulaire* (dioxane)
$\Delta\varepsilon = -0{,}77$ à 277 nm (maximum)
$\Delta\varepsilon = -12{,}5$ à 227 nm (maximum)
$\Delta\varepsilon = +7{,}23$ à 207 nm (maximum).

Exemple 12

1R, trans 2,2-diméthyl 3-(1',2'-dibromo 2',2'-dichloroéthyl) cyclopropane 1-carboxylate de R,S α-chloro 3-phénoxy benzyle.

On mélange 8 g de 3-phénoxy benzaldéhyde, 15,5 g de chlorure de l'acide (1R, trans) 2,2-diméthyl

3-(1',2'-dibromo 2',2'-dichloroéthyl) cyclopropane 1-carboxylique, tiédit pour obtenir un liquide homogène, refroidit à 20°C, introduit 0,150 g de chlorure de zinc anhydre, abandonne pendant 17 heures à 20°C, sous agitation et obtient le (1R,trans) 2,2-diméthyl 3-(1',2'-dibromo 2',2'-dichloroéthyl) cyclopropane 1-carboxylate de R,S $\alpha$-chloro 3-phénoxy benzyle brut.

Le spectre infrarouge de ce produit fait apparaître une absorption caractéristique à 1750 $cm^{-1}$ (caractéristique du carbonyle).

Le spectre de RMN permet de constater l'absence de proton aldéhydique.

Exemple 13

1R,cis 2,2-diméthyl 3-(2',2'-dibromovinyl)cyclopropane-1-carboxylate de RS $\alpha$-cyano 3-phénoxy benzyle.

Dans 50 cm3 d'acétonitrile anhydre, on dissout 5,5 g du dérivé $\alpha$-chloré brut obtenu à l'exemple 1, 0,8 g de cyanure de potassium anhydre, agite pendant 17 heures à 20°C, ajoute de l'eau, extrait au benzène et après les traitements habituels recueille 5,6 g de produit brut que l'on purifie par chromatographie sur gel de silice en éluant avec un mélange d'éther de pétrole (Eb. 35—75°C) et d'éther éthylique (9/1) et obtient 4,3 g de 1R cis 2,2-diméthyl 3-(2',2'-dibromovinyl)cyclopropane-1-carboxylate de RS $\alpha$-cyano 3-phénoxy benzyle.

Exemple 14

1R, cis 2,2-diméthyl 3-(2'2'-dibromovinyl) cyclopropane 1-carboxylate de RS $\alpha$-cyano 3-phénoxy benzyle.

De manière analogue à celle de l'exemple 13, au départ du dérivé $\alpha$-bromé obtenu à l'exemple 5, on obtient le 1R, cis 2,2-diméthyl 3-(2',2'-dibromovinyl) cyclopropane 1-carboxylate de de RS $\alpha$-cyano 3-phénoxy benzyle.

Exemple 15

1R,cis 2,2-diméthyl 3-(2',2'-dibromovinyl)cyclopropane-1-carboxylate de RS $\alpha$-cyano 3-phénoxy benzyle.

Dans 15 cm3 d'acétonitrile, on dissout 1,17 g de dérivé $\alpha$-fluoré brut obtenu à l'exemple 11; ajoute 0,2 g de cyanure de potassium, agite pendant 17 heures à 20°C, chromatographie le résidu sur gel de silice en éluant avec un mélange d'éther de pétrole et d'éther (9/1) et obtient 1 g de 1R,cis 2,2-diméthyl 3-(2',2'-dibromovinyl)cyclopropane-1R-carboxylate de RS $\alpha$-cyano 3-phénoxy benzyle.

Exemple 16

1R,trans 2,2-diméthyl 3-(2',2'-dibromovinyl)cyclopropane-1-carboxylate de RS $\alpha$-cyano 3-phénoxy benzyle.

Dans la solution de 1R.trans 2.2-diméthyl 3-(2'.2'-dibromovinyl)cyclopropane-1-carboxylate de RS $\alpha$-chloro 3-phénoxy benzyle obtenu à l'exemple 6 on introduit 90 cm3 d'acétonitrile, 1,5 g de cyanure de potassium, agite pendant 16 heures, ajoute de l'eau, extrait au benzène, puis après les traitements habituels, concentre à sec par distillation sous pression réduite.

Le résidu est chromatographié sur gel de silice en éluant par un mélange de benzène et de cyclohexane (6/4) et l'on obtient 7,5 g de 1R, trans 2,2-diméthyl 3-(2',2'-dibromovinyl)cyclopropane 1-carboxylate de RS $\alpha$-cyano 3-phénoxy benzyle.

Exemple 17

1R,cis 2,2-diméthyl 3-(2',2'-dichlorovinyl)cyclopropane-1-carboxylate de RS $\alpha$-cyano 3-phénoxy benzyle.

A une solution de 1R,cis 2,2-diméthyl 3-(2',2'-dichlorovinyl)cyclopropane-1-carboxylate de RS $\alpha$-chloro 3-phénoxy benzyle obtenu à l'exemple 7, on ajoute 80 cm3 d'acétonitrile, 1,5 g de cyanure de potassium, agite pendant 17 heures, ajoute de l'eau, extrait au benzène et après les traitements classiques, concentre à sec par distillation sous pression réduite, chromatographie le résidu sur gel de silice en éluant par un mélange de benzène et de cyclohexane (6/4) et obtient: 6,4 g de 1R,cis 2,2-diméthyl 3-(2',2'-dichlorovinyl)cyclopropane-1-carboxylate de RS $\alpha$-cyano 3-phénoxy benzyle.

Exemple 18

2,2-diméthyl 3R-(2'-méthyl 1'-propényl)cyclopropane-1R-carboxylate de RS $\alpha$-cyano 3-phénoxy benzyle.

Dans 50 cm3 d'acétonitrile, on dissout 3,09 g de 2,2-diméthyl 3R-(2'-méthyl 1'-propényl)cyclopropane-1R-carboxylate de RS $\alpha$-chloro 3-phénoxy benzyle brut obtenu à l'exemple 8, ajoute 1,1 g de cyanure de potassium, agite pendant 48 heures, ajoute de la glace, extrait au benzène, sèche sur sulfate de magnésium, filtre, et concentre à sec par distillation sous pression réduite. Le résidu (3,96 g) est chromatographié sur gel de silice en éluant par un mélange de cyclohexane et d'acétate d'éthyle (9/1) et obtient 0,48 g de 2,2-diméthyl 3R-(2'-méthyl 1'-propényl)cyclopropane-1R-carboxylate de RS $\alpha$-cyano 3-phénoxy benzyle.

*Spectre Infra-Rouge* (chloroforme)
Absorption à 1733 $cm^{-1}$ caractéristiques du carboxyle;
absorptions à 1487—1587 $cm^{-1}$, caractéristiques des noyaux aromatiques.

Exemple 19

2-(parachlorophényl)2-isopropyl acétate de RS $\alpha$-cyano 3-phénoxy benzyle.

A la solution de 2-(parachlorophényl)2-isopropyl acétate de RS $\alpha$-chloro 3-phénoxy benzyle obtenu à l'exemple 9, on ajoute 80 cm3 d'acétonitrile, 1,5 g de cyanure de potassium, agite pendant 72 heures à 20°C, ajoute de l'éther, élimine par filtration l'insoluble formé, concentre le filtrat à sec par distillation sous pression réduite, chromatographie le résidu sur gel de silice en éluant par un mélange de cyclohexane et d'acétone (95/5) et obtient 6,5 g de 2-(parachlorophényl)2-isopropyl acétate de RS $\alpha$-cyano 3-phénoxy benzyle.

Exemple 20

1R, cis 2,2-diméthyl 3-(2',2'-dibromovinyl) cyclopropane 1-carboxylate de RS $\alpha$-cyano cinnamyle

A 4,76 g de 1R, cis 2,2-diméthyl 3-(2',2'-dibromovinyl) cyclopropane 1-carboxylate de RS $\alpha$-chloro cinnamyle brut obtenu à l'exemple 10 dans 75 cm3 d'acétonitrile, on ajoute 1,5 g de cyanure de potassium, agite pendant 42 heures, ajoute de la glace, extrait au benzène, lave à l'eau, sèche sur sulfate de magnésium, filtre et concentre à sec par distillation sous pression réduite. Les 5,3 g de résidu sont chromatographiés sur gel silice, en éluant par un mélange de cyclohexane et d'acétate d'éthyle (9/1) et on obtient 1,76 g de 1R,cis 2,2-diméthyl 3-(2',2'-dibromovinyl)cyclopropane-1-carboxylate de RS $\alpha$-cyano cinnamyle.

| | | | | |
|---|---|---|---|---|
| *Analyse:* | $C_{18}H_{17}Br_2N\,O_2$ (439,16) | | | |
| Calculé: | C% 49,2 | H% 3,9 | Br% 36,4 | N% 3,2 |
| Trouvé: | 49,5 | 4,1 | 34,2 | 2,8. |

Exemple 21

(1R,trans) 2,2-diméthyl 3-(1',2'-dibromo 2',2'-dichloroéthyl) cyclopropane 1-carboxylate de (R) $\alpha$-cyano 3-phénoxy benzyle et (1R, trans) 2,2-diméthyl 3-(1',2'-dibromo 2',2'-dichloroéthyl) cyclopropane 1-carboxylate de (S) $\alpha$-cyano 3-phénoxy benzyle.

A 17,8 g de (1R, trans) 2,2-diméthyl 3-(1',2'-dibromo 2',2'-dichloroéthyl) cyclopropane 1-carboxylate de R,S $\alpha$-chloro 3-phénoxy benzyle brut, obtenu à l'exemple 12, dissous dans 200 cm3 de benzène, on ajoute 2 g de cyanure de potassium, laisse pendant 50 heures à 20°C, sous agitation, dilue à l'eau, extrait au benzène, concentre à sec, chromatographie le résidu sur gel de silice en éluant avec un mélange d'éther et d'éther de pétrole (1/9) et isole 0,63 g de (1R,trans) 2,2-diméthyl 3-(1',2'-dibromo 2',2'-dichloroéthyl) cyclopropane 1-carboxylate de (R) $\alpha$-cyano 3-phénoxy benzyle (composé A), puis 1,13 g de (1R,trans) 2,2-diméthyl 3-(1',2'-dibromo 2',2'-dichloroéthyl)cyclopropane 1-carboxylate de (S).$\alpha$-cyano 3-phénoxy benzyle (composé B).

Le spectre de R.M.N. du composé A présente les caractéristiques suivantes (deuterochloroforme):

Pics à 1,32—1,35 p.p.m. caractéristiques des hydrogènes des méthyles en position 2 du cyclopropyle;

Pics à 4,18 et 4,35 p.p.m. et 4,35 et 4,53 p.p.m. caractéristiques de l'hydrogène en position 1' de la chaîne latérale éthylique;

Pics à 6,37 p.p.m., caractéristique de l'hydrogène porté par le même carbone que le groupement nitrile.

Le spectre de R.M.N. du composé B présente les caractéristiques suivantes (deuterochloroforme):

Pics à 1,2—1,25—1,32 p.p.m. correspondant aux hydrogènes des méthyles en position 2 du cyclopropyle;

Pics à 4,21—4,38—4,37—4,53 p.p.m. attribués au proton en position 1' de la chaîne latérale éthylique;

Pic à 6,42 p.p.m. attribué au proton benzylique.

Exemple 22

Exemple de composition insecticide

On prépare un concentré émulsifiable en mélangeant intimement:

—isomère A du 1R,cis 2,2-diméthyl 3-(2',2'-dibromovinyl) cyclopropane-1-carboxylate d'$\alpha$-fluoro 3-phénoxy benzyle: 1 g

—Tween 80, 20 g

—Topanol A: 0,1 g

—xylène: 78,9 g

Etude de l'activité insecticide de l'isomère B du 1R,cis 2,2-diméthyl 3-(2',2'-dibromovinyl)cyclopropane-1-carboxylate d'$\alpha$-fluoro 3-phénoxy benzyle.

Effet létal sur mouche domestique:

Les insectes tests sont des mouches domestiques de sexes mélangés. On opère par application topique de 1 $\mu l$ de solution acétonique sur le thorax dorsal des insectes. On utilise 50 individus par traitement. On effectue le contrôle de mortalité vingt-quatre heures après traitement.

On effectue une série d'essais sans agent de synergie et une série d'essais avec synergie par le butoxyde de pipéronyle dans la proportion 10 parties de pipéronyle butoxyde pour 1 partie de produit à tester. On effectue le contrôle de mortalité vingt-quatre heures après traitement.

Les résultats expérimentaux obtenus, résumés dans le tableau suivant, sont exprimés en DL50 (dose létale 50) en nanogrammes par insecte.

| | DL50 en ng/insecte |
|---|---|
| Essais sans addition de synergiste | 9,2 |
| Essais avec addition de butoxyde de pipéronyle | 3,13. |

**Revendications**

1. Sous toutes leurs formes stéréoisomères ou sous forme de mélanges de stéréoisomères, les composés de formule générale $I_A$:

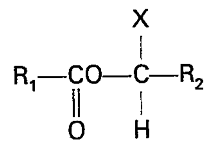

($I_A$)

dans laquelle:

—X représente un atome de fluore de chlore ou de brome,

— $R_1$ représente:

*soit* un groupement $R_1'$

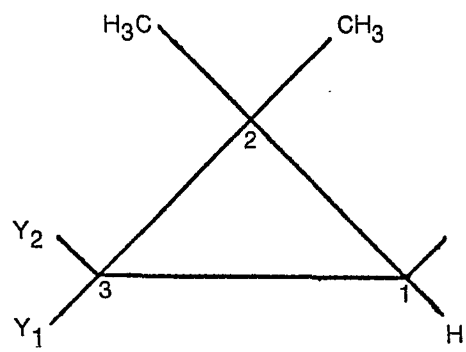

($R_1'$)

dans lequel

*ou bien* $Y_1$ et $Y_2$, sont identiques et représentent un radical méthyle,

*ou bien* $Y_1$ représente un atome d'hydrogène et $Y_2$ représente un groupement $Y_2'$:

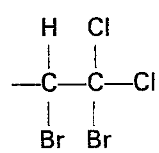

($Y_2'$)

*ou bien* $Y_1$ représente un atome d'hydrogène et $Y_2$ représente un groupement:

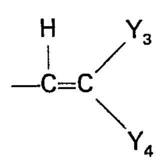

$Y_3$ et $Y_4$, identiques ou différents, représentant un atome de fluor, de chlore ou de brome, ou bien $Y_3$ et $Y_4$, identiques ou différents, représentant un radical méthyle ou un atome d'hydrogène, le groupement $R_1'$ correspondant, dans le cas où $Y_2$ représente un groupement:

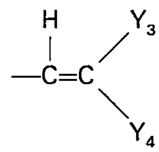

à un reste d'acide de structure cis ou trans, racémique ou optiquement actif, ou à un mélange de restes d'acides de structure cis et d'acide de structure trans,

soit un groupement ($R_1''$):

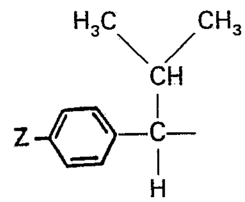

($R_1''$)

dans lequel Z représente un atome d'hydrogène, un atome de fluor, de chlore ou de brome, un radical alcoyle, linéaire ou ramifié, comportant de 1 à 4 atomes de carbone, un radical alcoyloxy, linéaire ou ramifié, comportant de 1 à 4 atomes de carbone, le groupement ($R_1''$) correspondant à un reste d'acide racémique ou optiquement actif,

— $R_2$ représente, dans le cas où $Y_2$ est différent de $Y_2'$:

*ou bien* un reste choisi parmi les groupements suivants:

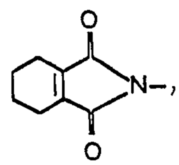, 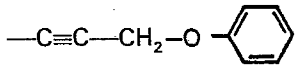, 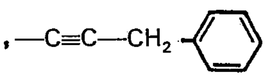,

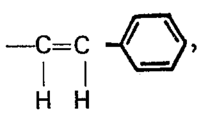, 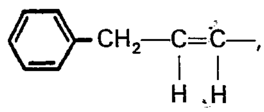, 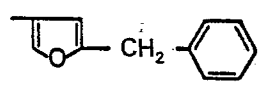

*ou bien* le reste 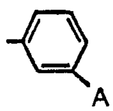 dans lequel A représente soit un groupement:

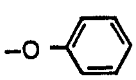 soit un groupement: 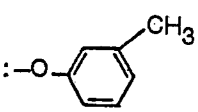 soit un groupement: 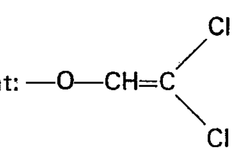

soit un groupement: 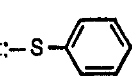 soit un groupement: 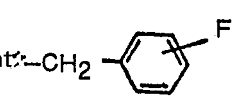

ou $R_2$ représente, dans le cas où $Y_2$ représente un groupe $Y_2'$, le reste

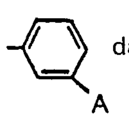 dans lequel A représente un groupement 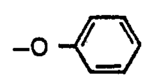

2. Sous toutes leurs formes stéréoisomères, les composés selon la revendication 1, de formule générale I:

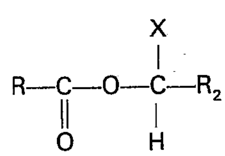

(I)

dans laquelle $R_2$ et X sont définis comme à la revendication 1, et R représente *soit* un groupement R':

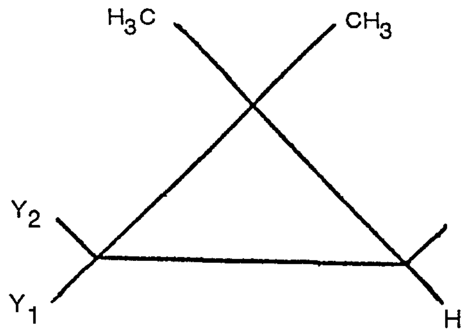

(R')

dans lequel:
*ou bien* $Y_1$ et $Y_2$, sont identiques et représentent un radical méthyle,
*ou bien* $Y_1$ représente un atome d'hydrogène et $Y_2$ représente un groupement:

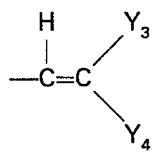

$Y_3$ et $Y_4$, identiques ou différents, représentant un atome de fluor, de chloro ou de brome, ou bien $Y_3$ et $Y_4$, identiques ou différents, représentant un radical méthyle ou un atome d'hydrogène, le groupement $R_1'$ correspondant, dans le cas où $Y_2$ représente un groupement:

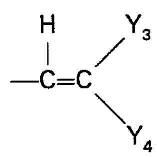

à un reste d'acide de structure cis ou trans, racémique ou optiquement actif, ou à un mélange de restes d'acide de structure cis et d'acide de structure trans.

—*soit* un groupement $R_1''$ tel que défini à la revendication 1.

3. Les composés de formule I, conformes à la revendication 2 comportant un groupement (R') dans lequel $Y_1$ représente un atome d'hydrogène et $Y_2$ représente un groupement:

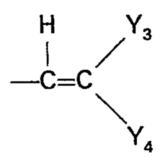

4. Les composés de formule I, conformes à la revendication 2, dans lesquels $R_2$ représente un groupement:

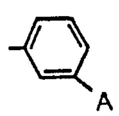 A ayant pour valeur: 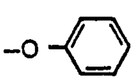

5. Les composés de formule I, conformes à la revendication 2, dans lesquels X représente un atome de fluor.

6. Les composés de formule I, conformes à l'une des revendications 2 ou 4, dans lesquels $R_2$ représente un groupement:

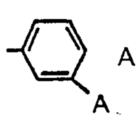 A ayant pour valeur: 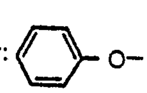

et dans lesquels X représente un atome de fluor ou de brome.

7. Les composés de formule I, conformes à la revendication 2, dans lesquels X représente un atome de chlore.

8. Les composés de formule générale I selon l'une quelconque des revendications 2 ou 4, dont les noms suivent:

—le 1R,cis 2,2-diméthyl 3-(2′,2′-dibromovinyl)cyclopropane-1-carboxylate de RS $\alpha$-chloro 3-phénoxy benzyle,

—le 1R,cis 2,2-diméthyl 3-(2′,2′-dibromovinyl)cyclopropane-1-carboxylate de RS $\alpha$-fluoro 3-phénoxy benzyle,

—le 1R,cis 2,2-diméthyl 3-(2′,2′-dichlorovinyl)cyclopropane-1-carboxylate de RS $\alpha$-chloro 3-phénoxy benzyle,

—le 2-(parachlorophényl)2-isopropyl acétate de RS $\alpha$-chloro 3-phénoxy benzyle.

9. Le composé de formule générale $I_A$ suivant:

—le 1R, trans 2,2-diméthyl 3-(1′,2′-dibromo 2′,2′-dichloroéthyl) cyclopropane 1-carboxylate de R,S $\alpha$-chloro 3-phénoxy benzyle.

10. Procédé de préparation des composés de formule générale $I_A$ tels que définis à la revendication 1, caractérisé en ce que l'on fait réagir, en présence d'un catalyseur acide, un halogénure d'acide de formule générale II:

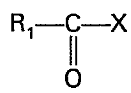 (II)

dans laquelle X et $R_1$ conservent les significations de la revendication 1, avec un aldéhyde de formule générale III:

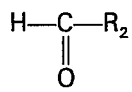 (III)

dans laquelle $R_2$ conserve les significations de la revendication 1.

11. Procédé de préparation, selon la revendication 10, des composés de formule générale I, tels que définis à la revendication 2, caractérisé en ce que l'on utilise au départ un halogénure d'acide de formule générale $II_A$:

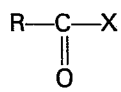 ($II_A$)

dans laquelle X et R sont définis comme à la revendication 2.

12. Procédé de préparation selon la revendication 10 ou 11, caractérisé en ce que le catalyseur est soit un acide de Lewis, tel que le chlorure de zinc, le chlorure d'aluminium ou le chlorure ferrique, soit un acide protonique tel que l'acide paratoluène sulfonique ou l'oléum.

13. Procédé de préparation des composés de formule générale I, tels que définis à la revendication 2, dans lesquels X représente un atome de fluor et dans lesquels $R_2$ représente soit un reste.

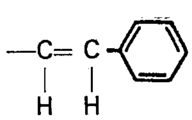 soit un reste: 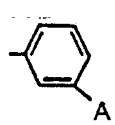

dans lequel A représente un groupement: 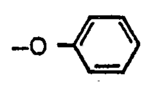

un groupement: 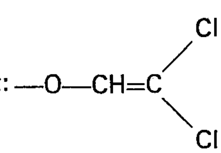 ou un groupement: 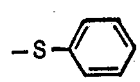

caractérisé en ce que l'on fati réagir un fluorure d'ammonium quaternaire fixé sur une résine avec un chlorure $Z_1$ soit de formule:

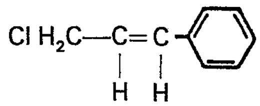 soit de formule: 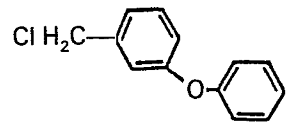

soit de formule: 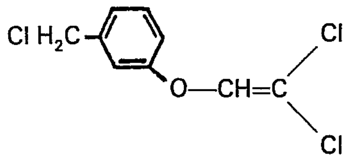

soit de formule: 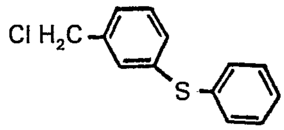

soumet, en présence, d'azoisobutyronitrile, le fluorure $Z_2$ résultant correspondant, à l'action du N-bromo succinimide pour obtenir un dérivé mixte bromé et fluoré $Z_3$ soit de formule:

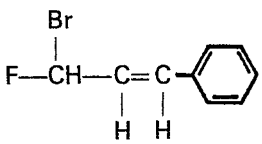 soit de formule: 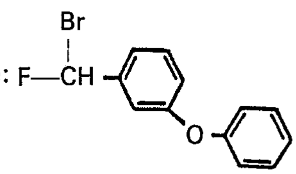

soit de formule: 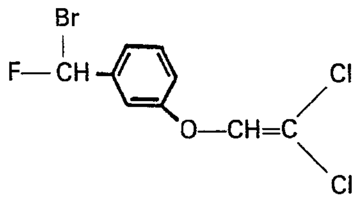

soit de formule: 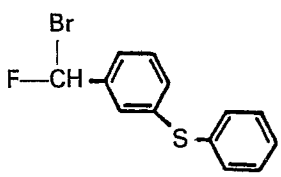

puis fait réagir ce composé avec un sol alcalin de formule générale IV:

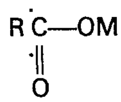 IV

dans laquelle R conserve les significations de la revendication 2 et M représente un ion dérivant d'un métal alcalin.

14. Application des composés de formule $I_A$, tels que définis à la revendication 1, sous toutes leurs formes stéréoisomères ou sous forme de mélanges de stéréoisomères, à la préparation, sous les formes stéréoisomères correspondantes, des composés de formule générale $I_B$:

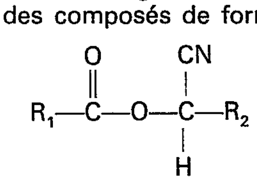 ($I_B$)

dans laquelle $R_1$ et $R_2$ sont définis comme à la revendication 1, application caractérisée en ce que l'on fait réagir un composé générateur d'ions $CN^-$ sur un composé de formule générale $I_A$:

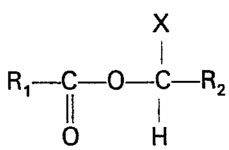 ($I_A$)

telle que définie à la revendication 1, les composés de formule $I_B$ résultants, existant sous les formes stéréoisomères correspondant aux formes stéréoisomères des composés $I_A$ utilisés.

15. Application, selon la revendication 14, des composés de formule I, tels que définis à la revendication 2, sous toutes leurs formes stéréoisomères, à la préparation, sous les formes stéréoisomères correspondantes, des composés de formule $I_C$:

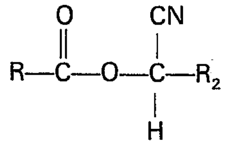

($I_C$)

dans laquelle R et $R_2$ sont définis comme à la revendication 2, application caractérisée en ce que l'on fait réagir un composé générateur d'ions $CN^-$ sur un composé de formule générale I.

16. Application, selon la revendication 14 ou 15, caractérisée en ce que, dans la formule $I_A$ ou I, X représente un atome de fluor ou de chlore.

17. Application, selon la revendication 14 ou 15, caractérisée en ce que la condensation avec l'agent générateur d'ions $CN^-$ est effectuée au sein d'un solvant organique anhydre, choisi dans le groupe constitué par l'acétonitrile et le diméthylformamide.

18. Application, selon la revendication 14 ou 15, caractérisée en ce que l'agent générateur d'ions $CN^-$ est un cyanure alcalin.

19. Application, selon l'une quelconque des revendications 15 à 18, à la préparation du 1R, cis 2,2-diméthyl 3-(2',2'-dibromovinyl) cyclopropane 1-carboxylate de RS $\alpha$-cyano 3-phénoxy benzyle, caractérisé en ce que le composé de formule I utilisé est le 1R, cis 2,2-diméthyl 3-(2',2'-dibromovinyl) cyclopropane 1-carboxylate de RS $\alpha$-chloro 3-phénoxy benzyle.

20. Application, selon l'une quelconque des revendications 15 à 18, à la préparation du 1R, cis 2,2-diméthyl 3-(2',2'-dibromovinyl) cyclopropane 1-carboxylate de RS $\alpha$-cyano 3-phénoxy benzyle, caractérisée en ce que le composé de formule I utilisé est le 1R, cis 2,2-diméthyl 3-(2',2'-dibromovinyl)cyclopropane 1-carboxylate de RS $\alpha$-fluoro 3-phénoxy benzyle.

21. Application, selon l'une quelconque des revendications 15 à 18, à la préparation du 1R, cis 2,2-diméthyl 3-(2',2'-dichlorovinyl) cyclopropane 1-carboxylate de RS $\alpha$-cyano 3-phénoxy benzyle, caractérisée en ce que le composé de formule I utilisé est le 1R, cis 2,2-diméthyl 3-(2'-dichlorovinyl) cyclopropane 1-carboxylate de RS $\alpha$-chloro 3-phénoxy benzyle.

22. Application, selon l'une quelconque des revendications 15 à 18, à la préparation du 2-parachlorophényl 2-isopropyl acétate de RS $\alpha$-cyano 3-phénoxy benzyle, caractérisée en ce que le composé de formule I utilisé est le 2-parachlorophényl 2-isopropyl acétate de RS $\alpha$-chloro 3-phénoxy benzyle.

23. Application, selon l'une quelconque des revendications 14 et 16 à 18, à la préparation du 1R, trans 2,2-diméthyl 3-(1',2'-dibromo 2',2'-dichloroéthyl) cyclopropane 1-carboxylate de RS $\alpha$-cyano 3-phénoxy benzyle, caractérisée en ce que le composé $I_A$ utilisé est le 1R, trans 2,2-diméthyl 3-(1',2'-dibromo 2',2'-dichloroéthyl) cyclopropane 1-carboxylate de RS $\alpha$-chloro 3-phénoxy benzyle ou le 1R, trans 2,2-diméthyl 3-(1',2'-dibromo 2',2'-dichloroéthyl) cyclopropane 1-carboxylate de RS $\alpha$-fluoro 3-phénoxy benzyle.

**Patentansprüche**

1. In sämtlichen ihrer stereoisomeren Formen oder in Form von Gemischen der Stereoisomeren die Verbindungen der allgemeinen Formel $I_A$,

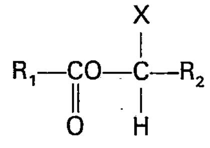

$I_A$

worin

X ein Fluor-, Chlor- oder Bromatom bedeutet, $R_1$ entweder eine Gruppe $R_1'$,

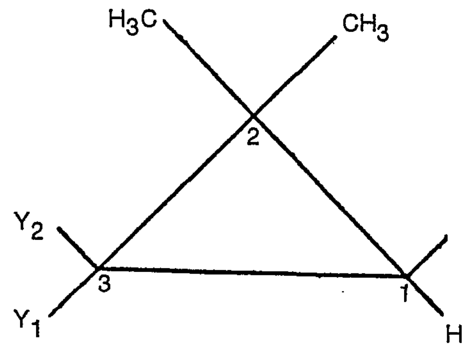

$R_1'$

worin entweder $Y_1$ und $Y_2$ gleich sind und einen Methylrest darstellen oder $Y_1$ ein Wasserstoffatom darstellt und $Y_2$ eine Gruppe $Y_2'$

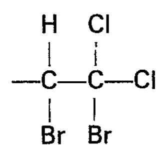

$Y_2'$

bedeutet oder $Y_1$ ein Wasserstoffatom bedeutet und $Y_2$ eine Gruppe

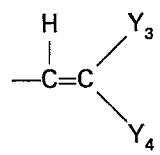

darstellt, worin $Y_3$ und $Y_4$, die gleich oder verschieden sein können, ein Fluor-, Chlor- oder Bromatom darstellen oder $Y_3$ und $Y_4$, die gleich oder verschieden sein können, einen Methylrest oder ein Wasserstoffatom bedeuten, wobei die Gruppe $R_1'$, wenn $Y_2$ eine Gruppe

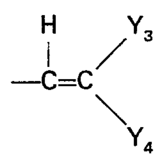

darstellt, einem Rest einer racemischen oder optisch aktiven Säure mit cis-oder trans-Struktur oder einem Gemisch von Resten der Säure mit cis-Struktur und der Säure mit trans-Struktur entspricht, oder eine Gruppe $R_1''$

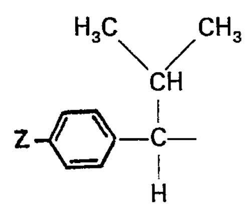

$R_1''$

darstellt, worin Z ein Wasserstoffatom, ein Fluor-, Chlor- oder Bromatom, einen linearen oder verzweigten Alkylrest mit 1 bis 4 Kohlenstoffatomen, einen linearen oder verzweigten Alkoxyrest mit 1 bis 4 Kohlenstoffatomen darstellt, wobei die Gruppe $R_1''$ einem Rest einer racemischen oder optisch aktiven Säure entspricht,
$R_2$, wenn $Y_2$ von $Y_2'$ verschieden ist,

entweder einen Rest, ausgewählt unter den folgenden Gruppen

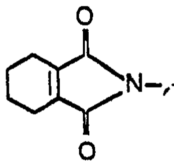, 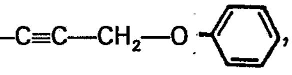, 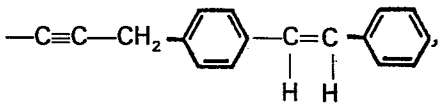, 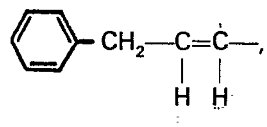,

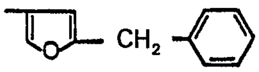 oder den Rest 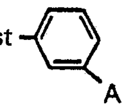 worin A entweder eine Gruppe

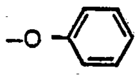 oder eine Gruppe 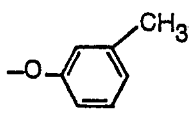 oder eine Gruppe 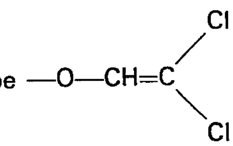

oder eine Gruppe 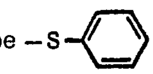 oder eine Gruppe 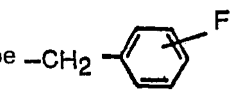

darstellt, bedeutet oder $R_2$, wenn $Y_2$ eine Gruppe $Y_2'$ darstellt,

den Rest 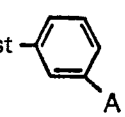 darstellt, worin A eine Gruppe 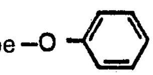 darstellt.

2. In ihren sämtlichen stereoisomeren Formen die Verbindungen gemäß Anspruch 1 der allgemeinen Formel I,

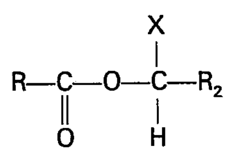 I

worin $R_2$ und X wie in Anspruch 1 definiert sind, und R entweder eine Gruppe R′,

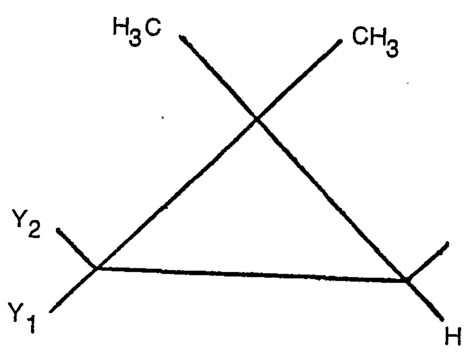 R′

worin $Y_1$ und $Y_2$ identisch sind und einen Methylrest bedeuten oder $Y_1$ ein Wasserstoffatom darstellt und $Y_2$ eine Gruppe

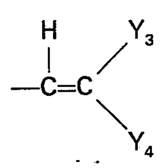

darstellt, wobei $Y_3$ und $Y_4$, die gleich oder verschieden sein können, ein Fluor-, Chlor- oder Bromatom bedeuten, oder $Y_3$ und $Y_4$, die gleich oder verschieden sein können, einen Methylrest oder ein Wasserstoffatom darstellen, wobei die Gruppe $R_1'$, wenn $Y_2$ eine Gruppe

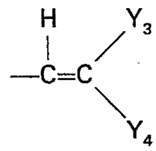

darstellt, einem Rest der racemischen oder optisch aktiven Säure mit cis- oder trans-Struktur oder einem Gemisch von Resten der Säure mit cis-Struktur und der Säure mit trans-Struktur entspricht, oder eine Gruppe $R_1''$ wie in Anspruch 1 definiert, bedeutet.

3. Verbindungen der Formel I gemäß Anspruch 2, die eine Gruppe R' enthalten, worin $Y_1$ ein Wasserstoffatom bedeutet und $Y_2$ eine Gruppe

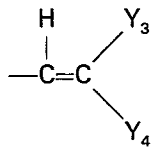

darstellt.

4. Verbindungen der Formel I gemäß Anspruch 2,

worin $R_2$ eine Gruppe 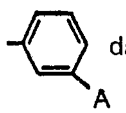 darstellt, wobei A die Gruppe 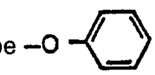 bedeutet.

5. Verbindungen der Formel I gemäß Anspruch 2, worin X ein Fluoratom bedeutet.

6. Verbindungen der Formel I gemäß einem der Ansprüche 2 oder 4, worin $R_2$ eine Gruppe

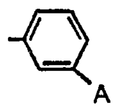

wobei A die Bedeutung 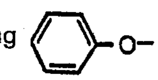 besitzt und worin X ein Fluor- oder Bromatom bedeutet, ist.

7. Verbindungen der Formel I gemäß Anspruch 2, worin X ein Chloratom bedeutet.

8. Die Verbindungen der allgemeinen Formel I gemäß einem der Ansprüche 2 oder 4 mit den folgenden Bezeichnungen:

RS-$\alpha$-Chlor-3-phenoxybenzyl-1R-cis-2,2-dimethyl-3-(2',2'-dibromvinyl)-cyclopropan-1-carboxylat,
RS-$\alpha$-Fluor-3-phenoxybenzyl-1R-cis-2,2-dimethyl-3-(2',2'-dibromvinyl)-cyclopropan-1-carboxylat,
RS-$\alpha$-Chlor-3-phenoxybenzyl-1R-cis-2,2-dimethyl-3-(2',2'-dichlorvinyl)-cyclopropan-1-carboxylat,
RS-$\alpha$-Chlor-3-phenoxybenzyl-2-(p-chlorphenyl)-2-isopropylacetat.

9. Die Verbindung der allgemeinen Formel $I_A$:
RS-$\alpha$-Chlor-3-phenoxybenzyl-1R-trans-2,2-dimethyl-3-(1',2'-dibrom-2',2'-dichloräthyl)-cyclopropan-1-carboxylat.

10. Verfahren zur Herstellung von Verbindungen der allgemeinen Formel $I_A$ gemäß Anspruch 1, dadurch gekennzeichnet, daß man in Gegenwart eines sauren Katalysators ein Säurehalogenid der allgemeinen Formel II

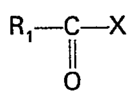

II

worin X und $R_1$ die in Anspruch 1 angegebenen Bedeutungen besitzen, mit einem Aldehyd der allgemeinen Formel III

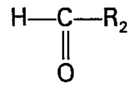

III

umsetzt, worin $R_2$ die in Anspruch 1 angegebene Bedeutung besitzt.

11. Verfahren gemäß Anspruch 10 zur Herstellung der Verbindungen der allgemeinen Formel I gemäß Anspruch 2, dadurch gekennzeichnet, daß man eine Säurehalogenid der allgemeinen Formel $II_A$

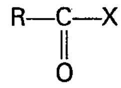 $II_A$

worin X und R wie in Anspruch 2 definiert sind, verwendet.

12. Herstellungsverfahren gemäß Anspruch 10 oder 11, dadurch gekennzeichnet, daß der Katalysator entweder eine Lewis-Säure wie Zinkchlorid, Aluminiumchlorid oder Ferrichlorid oder eine Protonensäure wie Paratoluolsulfonsäure oder Oleum ist.

13. Verfahren zur Herstellung der Verbindungen der allgemeinen Formel I gemäß Anspruch 2, worin X ein Fluoratom bedeutet und worin $R_2$ entweder einen Rest

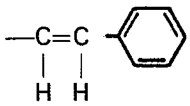 oder einen Rest 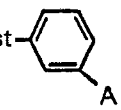 darstellt, worin A eine Gruppe 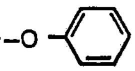

eine Gruppe 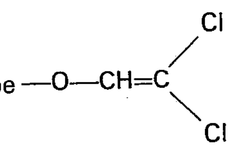 oder eine Gruppe 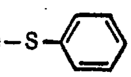

bedeutet, dadurch gekennzeichnet, daß man ein an einem Harz fixiertes quaternäres Ammoniumfluorid mit einem Chlorid $Z_1$ der Formel

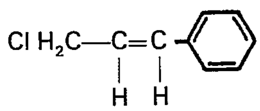 oder der Formel 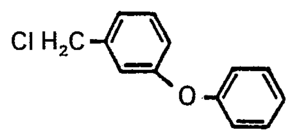

oder der Formel 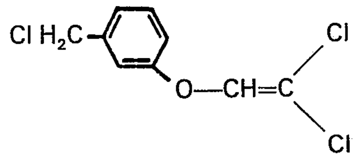

oder der Formel 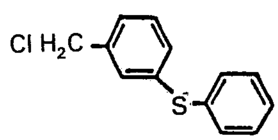

umsetzt, das erhaltene entsprechende Fluorid $Z_2$ der Einwirkung eines N-Bromsuccinimids unterzieht, um ein gemischtes bromiertes und fluoriertes Derivat $Z_3$ der Formel

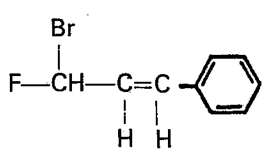 oder der Formel 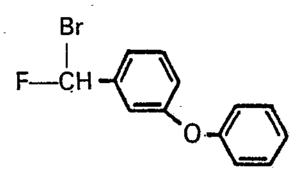

oder der Formel 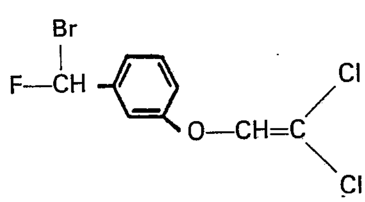

oder der Formel 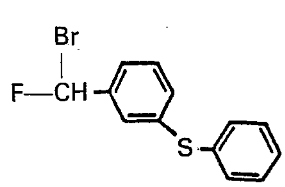

zu erhalten, anschließend diese Verbindung mit einem Alkalisalz der allgemeinen Formel IV

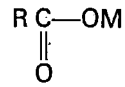

IV

umsetzt, worin R die in Anspruch 2 angegebenen Bedeutungen besitzt und M ein sich von einem Alkalimetall ablietendes Ion darstellt.

14. Verwendung der Verbindungen der Formel $I_A$ gemäß Anspruch 1 in sämtlichen ihrer stereoisomeren Formen oder in Form von Gemischen der Stereoisomer zur Herstellung in den entsprechenden stereoisomeren Formen der Verbindungen der allgemeinen Formel $I_B$

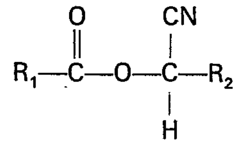

$I_B$

worin $R_1$ und $R_2$ wie in Anspruch 1 definiert sind, dadurch gekennzeichnet, daß man eine $CN^-$ -Ionen liefernde Verbindung mit einer Verbindung der allgemeinen Formel $I_A$

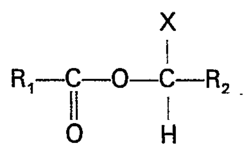

$I_A$

die wie in Anspruch 1 definiert ist umsetzt wobei die erhaltenen Verbindungen der Formel $I_B$ in den den stereoisomeren Formen der verwendeten Verbindungen $I_A$ entsprechenden stereoisomeren Formen vorliegen.

15. Verwendung gemäß Anspruch 14 der Verbindungen der Formel I gemäß Anspruch 2 in sämtlichen ihrer stereoisomeren Formen zur Herstellung in den entsprechenden stereoisomeren Formen der Verbindungen der Formel $I_C$

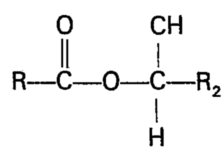

$I_C$

worin R und $R_2$ wie in Anspruch 2 definiert sind, dadurch gekennzeichnet, daß man eine $CN^-$-Ionen liefernde Verbindung mit einer Verbindung der allgemeinen Formel I umsetzt.

16. Verwendung gemäß Anspruch 14 oder 15, dadurch gekennzeichnet, daß in der Formel $I_A$ oder I X ein Fluor- oder Chloratom bedeutet.

17. Verwendung gemäß Anspruch 14 oder 15, dadurch gekennzeichnet, daß die Kondensation mit dem $CN^-$-Ionen liefernden Mittel in dem Medium eines wasserfreien organischen Lösungsmittels, ausgewählt unter Acetonitril und Dimethylformamid, durchgeführt wird.

18. Verwendung gemäß Anspruch 14 oder 15, dadurch gekennzeichnet, daß das $CN^-$-Ionen liefernde Mittel ein Alkalicyanid ist.

19. Verwendung gemäß einem der Ansprüche 15 bis 18 zur Herstellung von RS-$\alpha$-Cyano-3-phenoxybenzyl-1R-cis-2,2-dimethyl-3-(2',2'-dibromvinyl)-cyclopropan-1-carboxylat, dadurch gekennzeichnet, daß die verwendete Verbindung der Formel I das RS-$\alpha$-Chlor-3-phenoxybenzyl-1R-cis-2,2-dimethyl-3-(2',2'-dibromvinyl)-cyclopropan-1-carboxylat ist.

20. Verwendung gemäß einem der Ansprüche 15 bis 18 zur Herstellung von RS-$\alpha$-Cyano-3-phenoxybenzyl-1R-cis-2,2-dimethyl-3-(2',2'-dibromvinyl)-cyclopropan-1-carboxylat, dadurch gekennzeichnet, daß die verwendete Verbindung der Formel I das RS-$\alpha$-Fluor-3-phenoxybenzyl-1R-cis-2,2-dimethyl-3-(2',2'-dibromvinyl)-cyclopropan-1-carboxylat ist.

21. Verwendung gemäß einem der Ansprüche 15 bis 18 zur Herstellung von RS-$\alpha$-Cyano-3-phenoxybenzyl-1R-cis-2,2-dimethyl-3-(2',2'-dichlorvinyl)-cyclopropan-1-carboxylat, dadurch gekennzeichnet, daß die verwendete Verbindung der Formel I das RS-$\alpha$-Chlor-3-phenoxybenzyl-1R-cis-2,2-dimethyl-3-(2',2'-dichlorvinyl)-cyclopropan-1-carboxylat ist.

22. Verwendung gemäß einem der Ansprüche 15 bis 18 zur Herstellung von RS-$\alpha$-Cyano-3-phenoxybenzyl-2-p-chlorphenyl-2-isopropylacetat, dadurch gekennzeichnet, daß die verwendete Verbindung der Formel I das RS-$\alpha$-Chlor-3-phenoxybenzyl-2-p-chlorphenyl-2-isopropylacetat ist.

23. Verwendung gemäß einem der Ansprüche 14 und 16 bis 18 zur Herstellung von RS-$\alpha$-Cyano-

3-phenoxybenzyl-1R-trans-2,2-dimethyl-3-(1′,2′-dibrom-2′,2′-dichloräthyl)-cyclopropan-1-carboxylat, dadurch gekennzeichnet, daß die verwendete Verbindung der Formel $I_A$ das RS-$\alpha$-Chlor-3-phenoxy-benzyl-1R-trans-2,2-dimethyl-3-(1′,2′-dibrom-2′,2′-dichloräthyl)-cyclopropan-1-carboxylat oder das RS - $\alpha$ - Fluor - 3 - phenoxybenzyl - 1R - trans - 2,2 - dimethyl - 3 - (1′,2′ -dibrom - 2′,2′ - dichloräthyl)-cyclopropan - 1 - carboxylat ist.

**Claims**

1. In all their stereoisomeric forms or in the form of mixtures of stereoisomers, the compounds of general formula $I_A$:

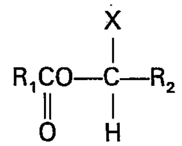

($I_A$)

in which:

—X represents a fluorine, chlorine or bromine atom,

—$R_1$ represents:

either a group $R_1'$:

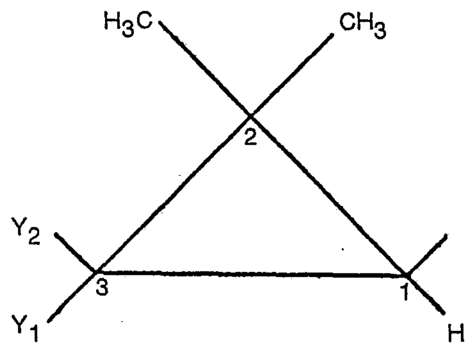

($R_1'$)

in which:

*either* $Y_1$ and $Y_2$ are identical and represent a methyl radical,

*or* $Y_1$ represents a hydrogen atom and $Y_2$ represents a group $Y_2'$:

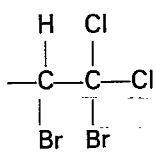

($Y_2'$)

*or* $Y_1$ represents a hydrogen atom and $Y_2$ represents a group:

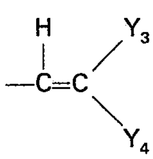

$Y_3$ and $Y_4$, being identical or different, representing a fluorine, chlorine or bromine atom or $Y_3$ and $Y_4$, being identical or different, representing a methyl radical or a hydrogen atom, the group $R_1'$ corresponding, in the case in which $Y_2$ represents a group:

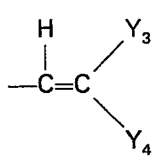

to a racemic or optically-active acid residue of *cis* or *trans* structure, or to a mixture of residues of acid of *cis* structure and of acid of *trans* structure, or a group $R_1''$:

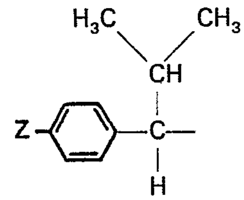

(R₁'')

in which Z represents a hydrogen atom, a fluorine, chlorine or bromine atom, a straight or branched alkyl radical containing from 1 to 4 carbon atoms or a straight or branched alkyloxy radical containing from 1 to 4 carbon atoms, the group $R_1''$ corresponding to a racemic or optically-active acid residue, and

—$R_2$ represents, in the case in which $Y_2$ is different from $Y_2'$:

*either* a residue selected from the following groups:

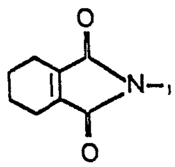, 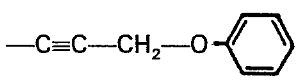 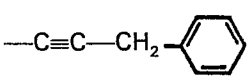 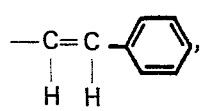,

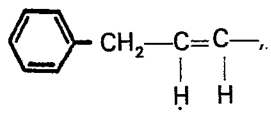, 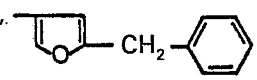

*or* the residue: 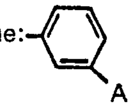 in which A represents either a group:

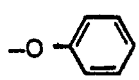 or a group: 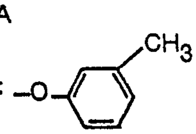 or a group: 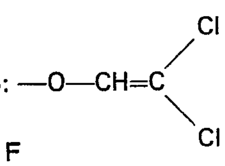

or a group: 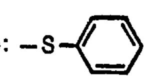 or a group: 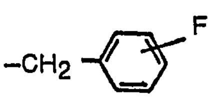

or $R_2$ represents, in the case in which $Y_2$ represents a group $Y_2'$, the residue

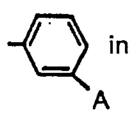 in which A represents a group 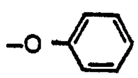

2. In all their stereoisomeric forms, the compounds according to Claim 1, of general formula I:

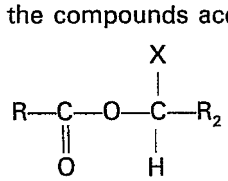

(I)

in which $R_2$ and X are defined as in Claim 1 and R represents:
*either* a group R':

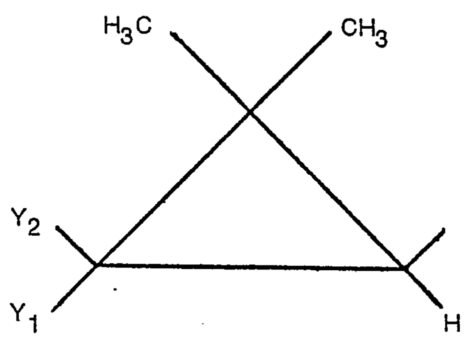

(R')

in which:
*either* $Y_1$ and $Y_2$ are identical and represent a methyl radical,
*or* $Y_1$ represents a hydrogen atom and $Y_2$ represents a group:

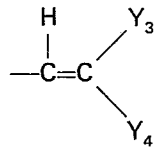

$Y_3$ and $Y_4$, being identical or different, representing a fluorine, chlorine or bromine atom, or $Y_3$ and $Y_4$, being identical or different, representing a methyl radical or a hydrogen atom, the group $R_1'$ corresponding, in the case in which $Y_2$ represents a group:

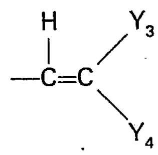

to a racemic or optically-active acid residue of *cis* or *trans* structure or to a mixture of residues of acid of *cis* structure and of acid of *trans* structure,
— *or* a group $R_1''$ as defined in Claim 1.

3. The compounds of formula I, according to Claim 2, containing a group R' in which $Y_1$ represents a hydrogen atom and $Y_2$ represents a group:

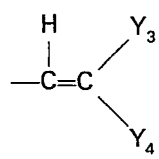

4. The compounds of formula I, according to Claim 2, in which $R_2$ represents a group:

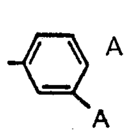 A having as value: 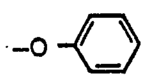

5. The compounds of formula I, according to Claim 2, in which X represents a fluorine atom.

6. The compounds of formula I, according to one of Claims 2 or 4, in which $R_2$ represents a group:

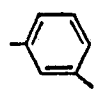 A having as value: 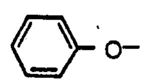

and in which X represents a fluorine or bromine atom.

7. The compounds of formula I, according to Claim 2, in which X represents a chlorine atom.

8. The compounds of general formula I, according to any one of Claims 2 or 4, of which the names are as follows:
—RS α-chloro 3-phenoxy benzyl 1R, *cis* 2,2-dimethyl 3-(2',2'-dibromovinyl) cyclopropane-1-carboxylate,
—RS α-fluoro 3-phenoxy benzyl 1R, *cis* 2,2-dimethyl 3-(2',2'-dibromovinyl) cyclopropane-1-carboxylate,
—RS α-chloro 3-phenoxy benzyl 1R, *cis* 2,2-dimethyl 3-(2',2'-dichlorovinyl) cyclopropane-1-carboxylate and
—RS α-chloro 3-phenoxy benzyl 2-(parachlorophenyl) 2-isopropyl acetate.

9. The following compound of general formula $I_A$:
—R,S α-chloro 3-phenoxy benzyl 1R, *trans* 2,2-dimethyl 3-(1',2'-dibromo 2',2'-dichloroethyl) cyclopropane-1-carboxylate.

10. Process for preparing the compounds of general formula $I_A$, as defined in Claim 1, characterised in that, in the presence of an acid catalyst, an acid halide of general formula II:

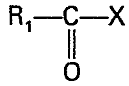 (II)

in which X and $R_1$ keep the meanings of Claim 1, is reacted with an aldehyde of general formula III:

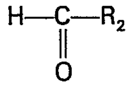 (III)

in which $R_2$ keeps the meanings of Claim 1.

11. Process for preparing, according to Claim 10, the compounds of general formula I, as defined in Claim 2, characterised in that to start with an acid halide of general formula $II_A$:

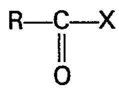 ($II_A$)

in which X and R are as defined as in Claim 2, is used.

12. Preparation process according to Claim 10 or 11, characterised in that the catalyst is either a Lewis acid such as zinc chloride, aluminium chloride or ferric chloride, or a protonic acid such as paratoluene sulphonic acid or oleum.

13. Process for preparing the compounds of general formula I, as defined in Claim 2, in which X represents a fluorine atom and in which $R_2$ represents either a residue:

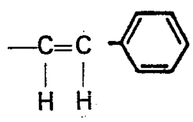 or a residue: 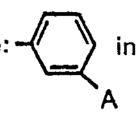 in which A represents a group: 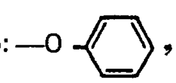,

a group: 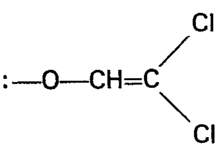 or a group: 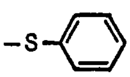

characterised in that a quaternary ammonium fluoride fixed on a resin is reacted with a chloride $Z_1$ either of formula:

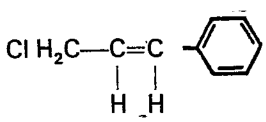 or of formula: 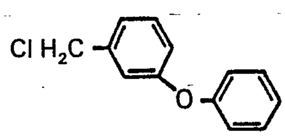

or of formula: 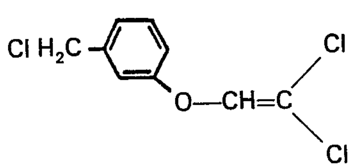

or of formula: 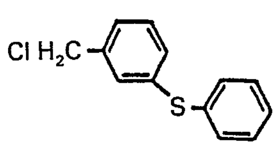

the corresponding resultant fluoride $Z_2$ is subjected, in the presence of azoisobutyronitrile, to the action of N-bromo succinimide to obtain a mixed brominated and fluorinated derivative $Z_3$ either of formula:

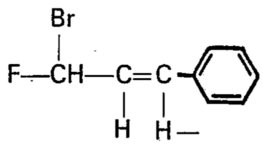 or of formula: 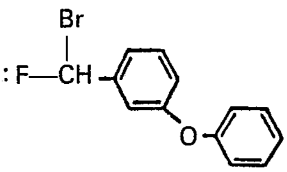

or of formula: 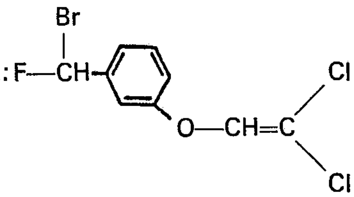

or of formula: 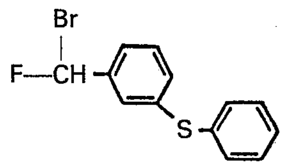

then this compound is reacted with an alkaline salt of general formula IV:

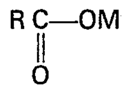 (IV)

in which R keeps the meanings of Claim 2 and M represents an ion derived from an alkali metal.

14. Use of the compounds of formula $I_A$, as defined in Claim 1, in all their stereoisomeric forms or in the form of mixtures of stereoisomers, in the preparation, in the corresponding stereoisomeric forms, of the compounds of general formula $I_B$:

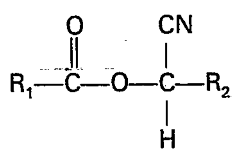 ($I_B$)

in which $R_1$ and $R_2$ are defined as in Claim 1, a use characterised in that a compound generating $CN^-$ ions is reacted with a compound of general formula $I_A$:

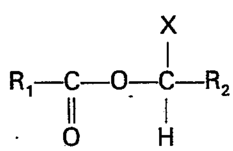 ($I_A$)

as defined in Claim 1, the resultant compounds of formula $I_B$ being in the stereoisomeric forms corresponding to the stereoisomeric forms of the compounds $I_A$ used.

15. Use according to Claim 14 of the compounds of formula I, as defined in Claim 2, in all their stereoisomeric forms, in the preparation, in the corresponding stereoisomeric forms, of the compounds of formula $I_C$:

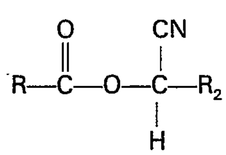 ($I_C$)

in which R and $R_2$ are defined as in Claim 2, a use characterised in that a compound generating $CN^-$ ions is reacted with a compound of general formula I.

16. Use according to Claim 14 or 15, characterised in that, in the formula $I_A$ or I, X represents a fluorine or chlorine atom.

17. Use according to Claim 14 or 15, characterised in that the condensation with the agent generating $CN^-$ ions is carried out in an anhydrous organic solvent selected from the group constituted by acetonitrile and dimethylformamide.

18. Use according to Claim 14 or 15, characterised in that the agent generating $CN^-$ ions is an alkali cyanide.

19. Use, according to any one of Claims 15 to 18, in the preparation of RS $\alpha$-cyano 3-phenoxy benzyl 1R, *cis* 2,2-dimethyl 3-(2′,2′-dibromovinyl) cyclopropane-1-carboxylate, characterised in that the compound of formula I used is RS $\alpha$-chloro 3-phenoxy benzyl 1R, *cis* 2,2-dimethyl 3-(2′,2′-dibromovinyl) cyclopropane-1-carboxylate.

20. Use, according to any one of Claims 15 to 18, in the preparation of RS $\alpha$-cyano 3-phenoxy benzyl 1R, *cis* 2,2-dimethyl 3-(2′,2′-dibromovinyl) cyclopropane-1-carboxylate, characterised in that the compound of formula I used is RS $\alpha$-fluoro 3-phenoxy benzyl 1R, *cis* 2,2-dimethyl 3-(2′,2′-dibromovinyl) cyclopropane-1-carboxylate.

21. Use, according to any one of Claims 15 to 18, in the preparation of RS $\alpha$-cyano 3-phenoxy benzyl 1R, *cis* 2,2-dimethyl 3-(2′,2′-dichlorovinyl) cyclopropane-1-carboxylate, characterised in that the compound of formula I used is RS $\alpha$-chloro 3-phenoxy benzyl 1R, *cis* 2,2-dimethyl 3-(2′,2′-dichlorovinyl) cyclopropane-1-carboxylate.

22. Use, according to any one of Claims 15 to 18, in the preparation of RS $\alpha$-cyano 3-phenoxy benzyl 2-parachlorophenyl 2-isopropyl acetate, characterised in that the compound of formula I used is RS $\alpha$-chloro 3-phenoxy benzyl 2-parachlorophenyl 2-isopropyl acetate.

23. Use, according to any one of Claims 14 and 16 to 18, in the preparation of RS $\alpha$-cyano 3-phenoxy benzyl 1R, *trans* 2,2-dimethyl 3-(1′,2′-dibromo 2′,2′-dichloroethyl) cyclopropane-1-carboxylate, characterised in that the compound $I_A$ used is RS $\alpha$-chloro 3-phenoxy benzyl 1R, *trans* 2,2-dimethyl 3-(1′,2′-dibromo 2′,2′-dichloroethyl) cyclopropane-1-carboxylate or RS $\alpha$-fluoro 3-phenoxy benzyl 1R, *trans* 2,2-dimethyl 3-(1′,2′-dibromo 2′,2′-dichloroethyl) cyclopropane-1-carboxylate.